(19) Europäisches Patentamt
European Patent Office
Office européen des brevets

(11) Publication number: **0 153 641**
**B1**

(12) # EUROPEAN PATENT SPECIFICATION

(45) Date of publication of patent specification: **26.10.88**

(51) Int. Cl.[4]: **G 01 N 31/22,** G 01 N 33/52, G 01 N 33/84, C 07 C 119/00

(21) Application number: **85101412.6**

(22) Date of filing: **11.02.85**

(54) **Ion test means having a porous carrier matrix.**

(30) Priority: **24.02.84 US 583127**

(43) Date of publication of application:
**04.09.85 Bulletin 85/36**

(45) Publication of the grant of the patent:
**26.10.88 Bulletin 88/43**

(84) Designated Contracting States:
**AT BE CH DE FR GB IT LI LU NL SE**

(56) References cited:
**EP-A-0 010 615**
**EP-A-0 041 175**
**EP-A-0 125 554**
**EP-A-0 125 555**
**GB-A-1 271 209**
**US-A-3 856 649**
**US-A-4 234 313**
**US-A-4 272 485**
**CLINICAL CHEMISTRY, vol. 28, no. 9, September 1982, pages 1857-1861, Washington, US; S.C. CHARLTON et al.: "Solid-phase colorimetric determination of potassium"**

(73) Proprietor: **MILES INC.**
**1127 Myrtle Street**
**Elkhart Indiana 46514 (US)**

(72) Inventor: **Gantzer, Mary Lou**
**25723 Kiser Court**
**Elkhart IN 46514 (US)**
Inventor: **Hemmes, Paul R. Jr.**
**1825 Brookwood Drive**
**Elkhart, IN 46514 (US)**
Inventor: **Wong, Daniel**
**1755 Spruce P1-D**
**Elkhart, IN 46514 (US)**

(74) Representative: **Dänner, Klaus, Dr. et al**
**c/o Bayer AG Konzernverwaltung RP**
**Patentabteilung**
**D-5090 Leverkusen 1 Bayerwerk (DE)**

(56) References cited:
**ANGEWANDTE CHEMIE, INTERNATIONAL EDITION, vol. 17, no. 11, 1978, pages 857-859, Weinheim, DE; J.P. DIX et al.: "Ion-selective crown ether dyes"**
**CHEMISCHE BERICHTE, vol. 109, no. 5, 1976, pages 1803-1831, Weinheim, DE; E. WEBER et al.: "Neue Kronenäther und ihre Alkalimetallion-Komplexe"**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 153 641 B1

**Description**

1. Introduction

The present invention relates to the measurement of ions, in particular ions in aqueous solution, and to a test means or device for performing such measurements. The invention provides a quick, facile way of assaying such ions whereby results are available to the analyst momentarily after merely contacting a test sample solution with the test means or device. There is no need for cumbersome, expensive electronic equipment such as ion-specific electrodes, flame photometers, atomic absorption spectrophotometers or the like. Nor is it necessary to resort to time-consuming wet chemistry techniques such as titration and other laboratory procedures. The present invention enables the analyst to merely contact the test sample with a strip device or similar test means configuration, and observe any detectable response.

The determination of aqueous ion concentration has application in numerous technologies. In the water purification art, calcium concentration must be carefully monitored to assess the degree of saturation of an ion exchange resin deionizer. Measurement of sodium and other ions in seawater is important in the preparation of drinking water aboard a ship at sea. Measurement of the potassium level in blood aids the physician in diagnosis of conditions leading to muscle irritability and excitatory changes in myocardial function. Such conditions include oliguria, anuria, urinary obstruction and renal failure due to shock. Measurement of lithium levels in the blood are particularly important since the toxic dose levels are only slightly higher than the therapeutic levels used in pyschiatric treatment.

Needless to say, a quick, facile method for determining ion concentration would greatly enhance the state of these technologies, as well as any others where such rapid, accurate determinations would be beneficial. Thus, for example, if a medical laboratory technician could accurately measure the sodium, lithium, potassium or calcium ion level of a serum or whole blood sample in a matter of seconds or minutes, such rapid results would aid the physician in diagnosis, and would increase laboratory efficiency manyfold.

2. Background of the Invention

Prior methods for determining ions in solution included flame photometry, atomic absorption photometry, ion-specific electrodes and test strip formats. The use of certain compounds and compositions which selectively isolate certain ions from a sample solution has become popular in ion-specific electrodes. These substances, known as ionophores, have the capability of selectively isolating ions from their counterions thereby causing a charge separation and a corresponding change in electrical conductivity in the phase containing the ionophore. Illustrative of the ion/ionophore phenomenon are ion assays utilizing membrane electrodes, liquid/liquid partitioning and fluorescence.

2.1 Ion-Specific Electrodes

When two solutions having different concentrations of ions are separated by an electrically conductive membrane, an electrical potential (EMF) is generated. The EMF developed by such a system is a function of concentration or ionic activity. This phenomenon is expressed mathematically by the well-known Nernst Equation

$$\varepsilon = \frac{RT}{nF} \ln \frac{\gamma_1 c_1}{\gamma_2 c_2} \qquad (1)$$

in which ε is the EMF of the particular system, F is the Faraday Constant [23,062.4 ± 0.003 calories (volt equiv.)$^{-1}$], R is the gas constant, T is the temperature in °C, ln is the natural logarithm and γ and c are, respectively, the activity coefficients and molal concentrations of the ion under study, the subscript 1 designates the solution on one side of the membrane, the subscript 2 denoting the solution on the other side, and n is the number of electrons transferred in the reaction.

In such membrane separation cells, the membrane can be a simple fritted glass barrier, allowing a small but measurable degree of ion diffusion from one solution to the other. Alternatively, a nonporous, electrically nonconductive film, such as polyvinyl chloride, impregnated with an ionophore can be employed. In the absence of the ionophore the film is an insulator and no DMF can be measured; when blended with an ionophore, charged ions are bound to the film and a small, measurable current can be induced to flow. Because the ionophore is selective in its affinity, and thus will bind only certain specific ions, such cells are ion selective. Any measureable EMF is due solely to the presence of those ions.

Thus, a cell for determining potassium ions ($K^+$) can be produced through use of an ionophore specific for $K^+$, e.g. valinomycin. In the presence of potassium, valinomycin produces a conductive path across a membrane by binding and transporting $K^+$, thus allowing a small current to flow. A reference concentration of $K^+$ is placed on one side of the membrane and the test sample on the other. The EMF developed is measured and used to calculate the unknown concentration from equation (1). Because $K^+$ binds to the valinomycin membrane, the conductive path only appears for $K^+$. Therefore, the EMF developed is attributable solely to the $K^+$ concentration gradient across the membrane.

The current flowing across the membrane is so small that no significant quantity of $K^+$ or counter-ion is transported through it. Electrical neutrality of the membrane is maintained either by a reverse flow of hydrogen ions, or by a parallel flow of $OH^-$ ions. This anion effect can reduce the specificity of the electrode towards the intended ion and is an interference to be minimized.

A major difficulty in the use of such ion-selective electrodes has been the marked reduction of accuracy and speed of response over time. Further, small changes in ion concentration produce such small changes in EMF that sophisticated voltmeter equipment is required.

It has also been shown that certain antibiotics, such as valinomycin, have an effect on the electrical properties of phospholipid bilayer membranes (biological membranes), such that these antibiotics solubilize cations within the membrane, in the form of mobile charged couples, thereby providing a "carrier" mechanism by which cations can cross the insulating hydrocarbon interior of the membrane. Such complexes have the sole purpose of carrying the charge of the complex through the membrane such that a voltage differential can be determined between solutions on either side of the membrane.

CH—A—479870 describes the use of porous membranes impregnated with macrocyclic derivatives of amino and oxy-acids in ion-selective electrodes. Materials used to form the membrane are glass frits and other porous membranes. Such electrodes are said to be effective in measuring ion activities.

US—A—4,053,381 issued to Hamblen, *et al.* discloses similar technology, and utilizes an ion specific membrane having ion mobility across it.

US—A—3,957,607 issued to Simon, *et al.* discloses a process for the electrochemical determination of cations utilizing an electrode having a membrane containing neutral ionophores capable of forming lipid soluble complexes with the cations.

2.2 Liquid/Liquid Partitioning

Another known application of ionophores in ion determination is through liquid/liquid partitioning. In this procedure, a hydrophobic ionophore is dissolved in an organic solvent immiscible with water. Eisenman *et al., J. Membrane Biol. 1*:294—345 (1969) disclose the selective extraction of cations from aqueous solutions into organic solvents via macrotetralide actin antibiotics. This technique involves merely shaking an organic solvent phase containing the antibiotics with aqueous solutions containing cationic salts of lipid-soluble colored anions, such as picrates and nitrophenolates. The intensity of color of the organic phase is then measured spectrophotometrically to indicate how much salt has been extracted. Phase transfer has also been studied by Dix et al., Angew. Chem. Int. Ed. Engl. 17:857 (1978) and in reviews including Burgermeister *et al., Top. Curr. Chem. 69*:91 (1977); Yu *et al.,* "Membrane Active Complexones," Elsevier, Amsterdam (1974); and Duncan, "Calium in Biological Systems," Cambridge University Press (1976).

Sumiyoshi, *et al., Talanta, 24,* 763—765 (1977) describes another method useful for determining $K^+$ in serum. In this technique serum is deproteinated by trichloroacetic acid, an indicator dye is added, and shaken with a solvent such as chloroform containing valinomycin.

Partitioning of a compound is rapid and effective between liquids, as shown by Eisenman, because of the mobility of the ionophore carrier and ions, which allows the transported species to diffuse rapidly away from the interface. Such a mechanism is normally impossible in the solid phase, because of the rigidity, immobility and essentially zero diffusion of materials in a solid phase.

2.3 Fluorescent Anions

Yet another approach to the measurement of ion activity in aqueous solutions utilizes fluorescent anions. [Feinstein, *et al., Proc. Nat. Acad. Sci. U.S.A., 68,* 2037—2041 (1971)]. It is stated that the presence of cation/ionophore complexes in organic solvents are known, but that complex formation in purely aqueous media had theretofore not been detected. Feinstein, *et al.,* demonstrated the existence of such complexes in water through the use of the fluorescent salts 1-anilino-8-naphthalene sulfonate and 2-*p*-toluidinyl sulfonate.

It was found that interaction of the ionophore/cation complexes with the fluorescent dyes produced enhanced fluorescence emission, increased lifetime and polarization, and significant blue-shift at the emission maxima of the fluorescence spectrum. At constant concentrations of ionophore and fluorophore, the intensity of fluorescence emission was found to be a function of cation concentration.

2.4 Chromophore-labeled Ionophore

An ion assay is disclosed in US—A—4,367,072 which makes use of a conjugate of an ionophore covalently bound to a chromophore material. In use, the conjugate is added to a liquid sample and the appearance of color in the solution is monitored spectrophotometrically.

The disclosure is limited to a solution assay, and it appears that insufficient color develops to enable direct visual observation. Moreover, the stoichiometric ratio of chromophore to ionophore is fixed in such a system due to the direct bonding between these molecules. Because of this direct bonding it is possible to regulate color intensity; the ratio of ionophore to chromophore is fixed.

2.5 Test Strip Format

EP—A—41 175 is directed to a nonporous carrier matrix incorporated with an ionophore. The carrier

may further be incorporated with a reporter substance, or the reporter substance may be added to the test sample. The ionophore (and reporter substance) are incorporated onto the nonporous carrier matrix as a film. The present invention does not incorporate a nonporous carrier matrix; to the contrary, the carrier matrix must, of necessity, be of a porous nature.

EP—A—125555 (cited under Act 54(3) EPC) is directed to a nonporous carrier matrix incorporated with an ionophore and a reporter substance. Accordingly, that disclosure differs from the present invention similarly as described in the preceeding paragraph.

EP—A—125554 (cited under Act 54(3) EPC) is directed to a hydrophilic carrier matrix incorporated with *finely divided globules* of a hydrophobic vehicle. The globules contain an ionophore and a reporter substance. They are formed by preparing an emulsion of the hydrophobic mixture and a mixture of water and a hydrophilic polymer such as gelatin. The emulsion is then coated onto a support member and the water evaporated leaving the hydrophilic polymer and the finely divided globules. The emulsion can be coated onto paper, evaporated and that carrier affixed to a support. In any case, the test means comprises *finely divided globules* of hydrophobic vehicle containing an ionophore and a reporter substance in a hydrophilic vehicle matrix. The present invention differs from the disclosure in the EP—A—125554 in that the ionophore and reporter substance are incorporated into a *porous* carrier matrix directly as a homogeneous hydrophobic composition. No emulsion is involved.

2.6 Summary

To summarize the background of technological developments leading up to the present invention, many methods are known for assaying ions in solution. Instrumental methods include such sophisticated techniques as ion-specific potentiometry, flame photometry and atomic absorption photometry. The use of ionophores which selectively complex with specific ions has lead to five basic approaches: ion selective electrodes, liquid/liquid partitioning, fluorescence enhancement, chromophore-labeled ionophore conjugates and test strips.

Unlike prior test formulations, the present invention provides a stable unitary test means (or device) by incorporating a homogeneous hydrophobic composition onto a porous carrier matrix.

3. Brief Description of the Drawings

Performance data depicting the dose response of a test device responsive to the presence of potassium ion is portrayed graphically in Figure 1. Data were taken using test devices formulated according to the procedure described in Example 11.5. The graph shows the calculated K/S values plotted versus concentration of potassium ion [$K^+$], in millimoles per liter or millimolar (mM). The formulation for a potassium responsive test device is a preferred embodiment of the invention disclosed herein.

Figure 2 shows reflectance spectra obtained 30 seconds after dipping test devices prepared as described in Example 11.2 in urine containing 30 to 120 millimolar (mM) sodium ion. The small change in the spectrum for devices subjected to the stress of 1 month storage at 40°C (dashed line) over the room temperature spectrum (solid line) indicates the stability to temperature stress of the sodium responsive device on a paper matrix formulated with the inclusion of buffer. Percent reflectance (%R) is plotted versus wavelength ($\lambda$) in nanometers (nM) for the sodium responsive test devices.

4. Summary of the Invention

The present invention resides in the discovery of a new test means for detecting the presence of an ion in an aqueous test sample and determining its concentration. The test means comprises a porous carrier matrix substantially uniformly incorporated with a) a homogeneous hydrophobic mixture containing three principal ingredients: an ionophore capable of forming a complex with the ion, a hydrophobic vehicle, and a reporter substance capable of interacting with the complex of ionophore and ion to produce a detectable response; and b) a buffering substance capable of providing a pH in the range of from about 5 to 10.

A test device comprises the test means affixed to one upper flat face of an elongated support member.

In use, the aqueous test sample is contacted with the test means or device. The presence and/or concentration of the ion in the test sample is then determined by observing any detectable response produced.

The test means and device of the present invention provide rapid results, sufficient detectable response forming in most instances in at least a few minutes.

5. Definitions

The following definitions are provided to clarify the scope of the present invention, and to enable its formulation and use.

5.1 The expression "ionophore" includes molecules capable of forming a complex with a particular ion, in some instances to the substantial exclusion of others. For example the cyclic polyether 2,3-naphtho-1,4,7,10,13-pentaoxacyclopentadeca-2-ene (sometimes known as 2,3-naphtho-15-crown-5 and called Potassium Ionophore I herein) binds selectively to potassium ions in solution to form a cationic complex. Included in the term are coronands, cryptands and podands.

5.2 A "reporter substance" is one which is capable of interacting with an ionophore/ion complex to produce a detectable response such as a color change. Thus, the reporter can be a dye capable of

interacting with the ionophore/ion complex causing the reporter to lose a proton and become charged, effecting a change in electron distribution. The change in electron distribution produced a detectable response. The expression "reporter substance" includes phenolic compounds, such as *p*-nitrophenol, which are relatively colorless in the nonionized state, but which color upon ionization, and fluorescent compounds which produce more or less fluorescence upon a change in electron distribution. The reporter substance can also be one which can trigger a detectable response together with other components. For example, the change in electron distribution in the reporter substance caused by interaction with the complex can in turn facilitate the interaction of the reporter with another component which would then produce a detectable response.

5.3 By "interacting" is meant any coaction between a reporter substance and an ionophore/ion complex which leads to a detectable response.

5.4 The expression "detectable response" is meant herein as a change in or occurrence of a parameter in a test means system which is capable of being perceived, either by direct observation or instrumentally, and which is a function of the presence of a specific ion in an aqueous test sample. Some detectable responses are the change in or appearance of color, fluorescence, reflectance, pH, chemiluminescence and infrared spectra.

5.5 By the expression "intermediate alkyl" as used herein is meant an alkyl group having from 5 to 15 carbon atoms. It includes normal and branched isomers. It can be unsubstituted or it can be substituted, provided any such substitution not interfere with the operation of the presently claimed test means or device in its capability to detect ions.

5.6 The expression "lower alkyl', as used in the present disclosure is meant as an alkyl moiety containing 1—4 carbon atoms. Included in the meaning of lower alkyl are methyl, ethyl, *n*-propyl, isopropyl, *n*-butyl, *sec*-butyl and *tert*-butyl. These can be unsubstituted, or they can be substituted provided any such substituents not interfere with the operation or functioning of the presently claimed test means or device in its capability to detect ions.

5.7 By "pseudohalogen" is meant atom or groups of atoms which, when attached to an unsaturated or aromatic ring system, affect the electrophilicity or nucleophilicity of the ring system, and/or have an ability to influence the distribution of an electrical charge through delocalization or resonance, in a fashion similar to the halogens. Thus, whereas halogen signifies Group VII atoms such as F, Cl, Br and I, pseudohalogens embrace the following moieties —CN, —SCN, —OCN, $—N_3$, —COZ, —COOZ, —CONHZ, $—CF_3$, $—CCl_3$, $—NO_2$, $—SO_2CF_3$, $—SO_2CH_3$, $—SO_2—C_6H_5$, $—SO_2C_6H_4CH_3$, $—SOC_6H_5$ and $—SOCF_3$ in which Z is alkyl or aryl.

5.8 The expression "porous" as used herein refers to the availability of interstices in the carrier matrix which allow an aqueous test sample ready access to the hydrophobic composition containing the ionophore and the reporter substance. For example, paper is a porous carrier matrix which maintains an open lattice structure even after it has been incorporated with the homogeneous hydrophobic composition and with the buffering substance and dried. Upon contact with the doubly dried and incorporated paper, the aqueous sample flows readily into that open network. The area of contact between the hydrophobic composition and the aqueous sample is accordingly very large.

5.9 By "homogeneous" is meant uniform dispersion of the composition throughout such that any randomly selected portion would contain the same amount of each of the composition ingredients.

5.10 The homogeneous hydrophobic composition is incorporated with the porous carrier matrix without any intentional discontinuties. However the porous nature of the matrix is maintained even after incorporation. Therefore the expression "substantially uniformly" is used in recognition of the fact that the matrix retains openings after drying into which an aqueous test sample may flow when contacted by the test means (or device).

6. Test Means

The present test means comprises three basic elements: (a) a porous carrier matrix; (b) a homogeneous hydrophobic composition containing an ionophore, a hydrophobic vehicle and a reporter substance; and (c) a buffering substance. When an aqueous test sample contains an ion capable of complexing with the ionophore, the ion can interact with the ionophore contained in the homogeneous hydrophobic composition producing an ionophore/ion complex, which in turn interacts with the reporter substance to produce a detectable response. A buffering substance which is incorporated directly into the porous carrier matrix provides the proper pH for the interaction of the ionophore/ion complex with the reporter substance. Because the test means is incorporated with the buffer, manipulation of the test sample to adjust the pH is unnecessary.

An interferent removal system and a wetting substance can be added to the test means (or device). Other components including stabilizers, preservatives thickeners [such as polyvinylmethyl ether (available commercially as Gantrez® M—154 from GAF Corp., New York, New York), polystyrene sulfonic acid or agarose], photochemical stabilizers and so forth can be added, provided they do not interfere with the production of the detectable response. Given the present disclosure, the choice of such components is well within the skill of those knowledgeable in the art. Any component added to the hydrophobic composition should not change its essential homogeneous hydrophobic character. Additionally, the carrier matrix should retain its porous nature after incorporating all the components and drying.

The composition of the present invention can be incorporated with the carrier matrix in a variety of ways. The ingredients can be dissolved in a suitable solvent or a mixture of solvents as described herein. Such a solution can be used to impregnate the carrier matrix by immersion or as an ink wherein the reagents are printed on a suitable matrix, or the carrier matrix can be coated with the composition, such as with a doctor blade. The preferred method is described in Section 10.1 under general procedure.

6.1 Carrier Matrix

The carrier matrix with which the homogeneous hydrophobic composition is incorporated must be able to support a hydrophobic phase in such a way that substantial openings exist after drying into which an aqueous test sample can easily move, i.e., it must be porous.

Suitable materials include paper, wood and other cellulosic systems, sintered ceramic frits, and porous polymeric materials provided that the dimensional integrity of the matrix is maintained upon incorporation of the hydrophobic composition and subsequent contact with the aqueous sample. In addition, the matrix material cannot interact with the hydrophobic composition in a way which would interfere with the production of a detectable response.

A preferred carrier matrix is paper. For example, filter paper can be incorporated with a homogeneous hydrophobic composition and dried. The paper can also be incorporated with a buffering substance. Upon contacting the test means with an aqueous test sample, the ion can easily reach the hydrophobic phase by flowing into the open lattice of the paper. Since the buffer is incorporated directly with the test means, no sample dilution is necessary to provide the property pH.

6.2 Hydrophobic Vehicle

The primary function of the hydrophobic vehicle is to increase the detectable response of the test means system. Thus the vehicle can be a liquid or a solid provided that it increases the ability of the ionophore/ion complex and reporter substance to coexist in the homogeneous hydrophobic composition. Care must be exercised to choose a vehicle which does not interfere with the interaction of the ionophore/ion complex and the reporter substance.

Substances which are useful as hydrophobic vehicles include liquids which are insoluble in basic solutions and are capable of dissolving an ionophore and a reporter substance. Preferably they are relatively nonvolatile, i.e., have a boiling point of at least about 150°C, ideally at least about 200°C. The vehicle is normally an oxygen donor, containing functional groups such as ether, ester, amide and the like or combinations thereof.

Typical liquid which fall into this category are tricresyl phosphate, dioctyl phthalate, *tris*-2-ethylhexyl phosphate, di-2-ethylhexyl sebacate, *n*-butyl-acetyl-ricinolate and nitrophenyl ethers such as 2-nitrophenyl octyl ether, 2-nitrophenyl butyl ether, dibenzyl ether and *o*-nitrophenyl 2-(1,3,3)-trimethyl butyl-5,7,7-triethyloctyl ether. Mixtures of these liquids can be used. Useful solids include *cis*-N,N,N',N'-tetraisobutyl-1;2-cyclohexane dicarboxamide and N,N'-diheptyl-5,5-dimethyl-N,N'-di(3-oxapentyl)-3,7-dioxanonane diamide.

It is possible for one compound in the homogeneous hydrophobic composition to serve as both the ionophore and the hydrophobic vehicle. For example *cis*-N,N,N',N'-tetraisobutyl-1,2-cyclohexane dicarboxamide (herein referred to as CDA)

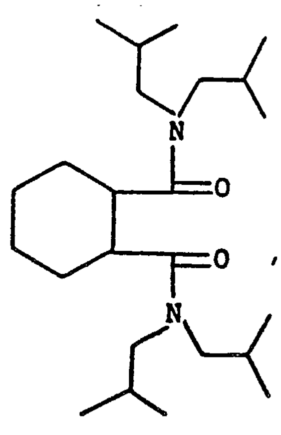,

was originally used in formulations for a lithium ion test as the hydrophobic vehicle. Later it was discovered that CDA alone would function as both the ionophore and hydrophobic vehicle.

6.3 Ionophores

The ionophore element of the present invention is a concept which is broad in scope, as characterized by the definition of the term in paragraph 5.1, *supra.* It includes multidentate cyclic compounds which contain donor atoms in their cyclic chains. Such multidentate cyclic compounds can be monocyclic or

polycyclic. Alternatively, the ionophore can be an open chain containing donor atoms. Thus, included in the ionophore element are monocyclic systems which are ion-specific, termed coronands; polycyclic ion-specific compounds known as cryptands; and open chain structures, which are known as podands, capable of selectively complexing with ions.

6.3.1 Coronands

Coronands are monocyclic compounds which contain donor atoms which are electron rich or deficient and which are capable of complexing with particular cations and anions because of their unique structures. Included in this term are crown ethers in which the monocyclic chain contains oxygen as the donor atom. Other coronands are compounds which contain an assortment of electron rich atoms such as oxygen, sulfur and nitrogen. Because of the unique sizes and geometries of particular coronands, they are adaptable to complexing with various ions. In so complexing, the electron rich atoms, such as the oxygens in a crown ether, orient towards the electron deficient cation. The carbon atom segments of the chain are simultaneously projected in a direction outwards from the ion. Thus, the resultant coronand/ion complex is charged in the center, but is hydrophobic at its perimeter.

6.3.2 Cryptands

Cryptands are polycyclic analogues of coronands. Accordingly, they include bicyclic and tricyclic multidentate compounds. The cyclic arrangement of donor atoms is three dimensional in space, as opposed to the substantially planar configuration of coronands. A cryptand is capable of virtually enveloping the ion in three dimensional fashion and, hence, is capable of strong bonds to the ion in forming the complex. Like in the coronands, the donor atoms can include such atoms as oxygen, nitrogen and sulfur.

6.3.3 Podands

Ions can also form complexes with noncyclic compounds. For example, a linear chain which contains a regular sequence of electron rich atoms such as oxygen has the capability of assocating with positively charged ions to form complexes, not entirely unlike the coronands and cryptands. The main structural difference between podands and the other two ionophores in the openness of the structure. Thus, podands can be subcategorized into monopodands, dipodands, tripodands, etc. A monopodand, therefore, is a single organic chain containing donor atoms, a dipodand is two such chains coupled by a bridge atom or group of atoms capable of variable spacial orientation, and a tripodand is three chains attached to a central atom or group of atoms. Simon, *et al.* in U.S. Patent No. 3,957,607 discloses dipodands particularly suited to the determination of calcium or barium ions. In the present invention, a preferred ionophore is the tripodand 1,1,1-*tris*[1'-(2'-oxa-4'-oxo-5'-aza-5'-methyl)dodecanyl]propane referred to herein as Sodium Ionophore I, which was found to be particularly useful in a test means for the determination of sodium ion. In fact, Sodium Ionophore I is 90 times more selective for sodium ions than the dipodand, N,N'-dibenzyl-N,N'-diphenyl-1,2-phenylenedioxydiacetamide. [Guggi, M., Oehme, M., Pretsch, E. and Simon, W. *Helv. Chim. Acta, 59*:2417 (1976)].

6.3.4 Specific Ionophores

Some of the ionophores which have been found especially useful when used in the instant invention are tabulated below, along with the cations with which they are capable of selectively complexing.

Chemical names for preferred ionophores follow with their structures. Common names assigned for use herein are noted in brackets.

| Ionophore | Cation |
|---|---|
| 1,1,1-*tris*[1'-(2'-oxa-4'-oxo-5'-aza-5'-methyl)dodecanyl]propane | $Na^+$ |

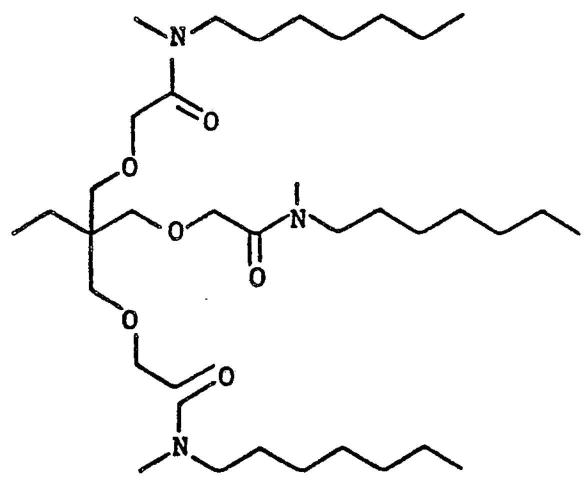

[Sodium Ionophore I ]

| Ionophore | Cation |
|---|---|
| N,N'-dibenzyl-N,N'-diphenyl-1,2-phenylenedioxydiacetamide | $Na^+$ |

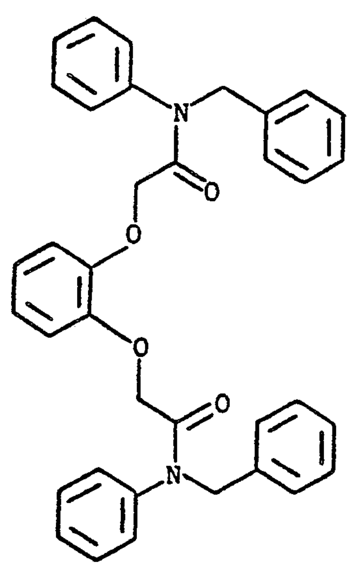

[Sodium Ionophore II]

6,7,9,10,18,19-hexahydro-17-*n*-butyl-dibenzo[b,k][1,4,7,10,13]pentaoxa-cyclohexadecine-18-yl-oxyacetic acid — $Na^+$

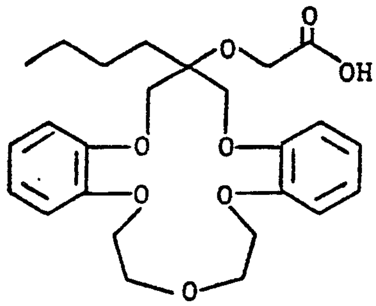

[Sodium Ionophore III]

| Ionophore | Cation |
|---|---|
| 2,3-naphtho-1,4,7,10,13-pentaoxacyclo-pentadeca-2-ene | $K^+$ |

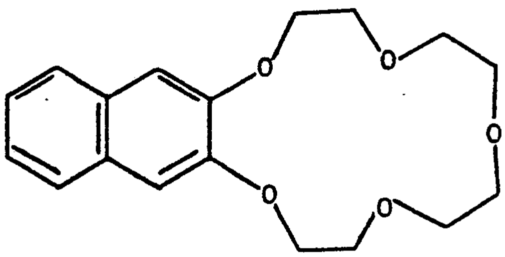

[Potassium Ionophore I]

N,N'-diheptyl-N,N',5,5-tetramethyl-3,7-dioxanonane diamide — $Li^+$

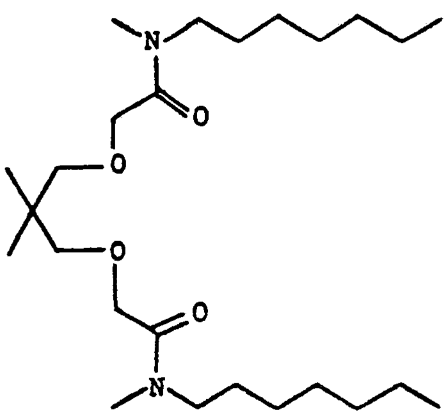

[Lithium Ionophore I]

| Ionophore | Cation |
|---|---|
| N,N'-diheptyl-5,5-dimethyl-N,N'-di(3-oxapentyl)-3,7-dioxanonane diamide | $Li^+$ |

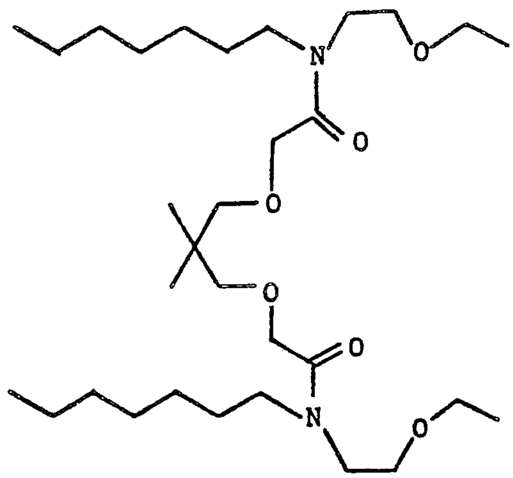

[Lithium Ionophore II]

| | |
|---|---|
| diethyl-N,N'-[(4R,5R)-4,5-dimethyl-1,8-dioxo-3,6-dioxaoctamethylene]*bis*(12-methylaminododecanoate) | $Ca^{++}$ |

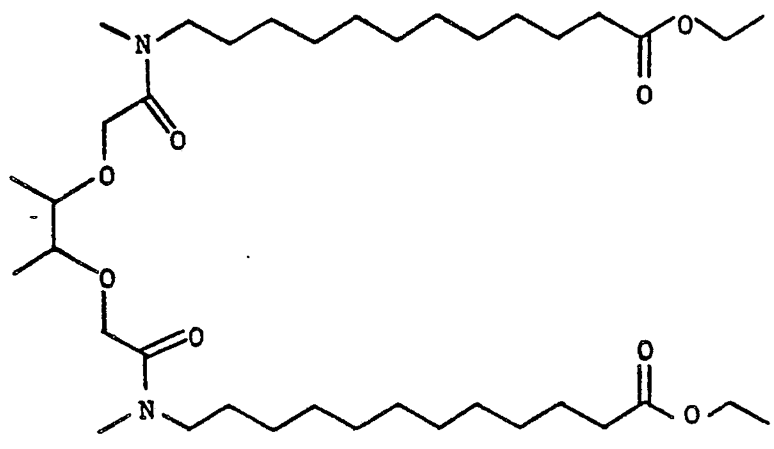

[Calcium Ionophore]

Other ionophores which are useful in the present invention include those listed below:

| Ionophore | Cation |
|---|---|
| 15-crown-5 | $Na^+$, $K^+$ |
| Valinomycin | $K^+$ |
| 4,7,13,16,21,24-hexaoxa-1,10-diazabicyclo [8,8,8]hexacosane (Kryptofix® 222) | $K^+$ |
| Dibenzo-18-crown-6 | $K^+$ |
| Dicyclohexano-18-crown-6 | $K^+$ |
| 4,7,13,18-tetraoxa-1,10-diaza-bicyclo-[8,5,5]eicosane (Kryptofix® 211) | $Li^+$ |
| 12-crown-4 | $Li^+$ |

Kryptofix® is a trademark of E. Merck, Darmstadt, West Germany.

Although these specific ionophores were used advantageously in the test means of the present invention, other ionophores or mixtures thereof, can also be used. In particular, ionophores which contain ionizable groups, such as Sodium Ionophore III, can be substituted in the formulation, so long as they have sufficient analyte-ion specificity.

6.4 Reporter Substance

Given the presence of the ion of interest in the test solution it is the reporter substance which provides the detectable response by interacting with the ionophore/ion complex. The reporter substance can range in composition from a single compounds, which can ionize in response to the formation of the ionophore/ion complex, to a mixture of reactive species which produce a detectable product when their reaction chain is triggered by the presence of the complex. Thus, it can be seen that when no analyte ion is present the reporter substance remains dormant; no detectable response is observed. Alternatively, when the particular ion under surveillance is present a complex is formed which interacts with the reporter substance and induces it to undergo a detectable change.

In the case where the reporter is a single compound, it can include a dissociable group such that upon dissociation a colored ionic species is formed. For example, phenolic compounds such as *p*-nitrophenol, are relatively colorless in the non-ionized state but are colored upon ionization. Other compounds, such as those which produce more or less fluorescence upon a change in electron distribution, can also be used. Glasses of fluorescent indicators and their derivatives which are useful in the present invention include derivatives of fluorescein, especially fluorescein esters, 7-hydroxy coumarins, resorufins, pyren-3-ols and flavones.

The reporter substance can also be one which can trigger a detectable response together with other components. For example a reaction system useful as the reporter substance is one which involves the dissociation of a proton from a phenol, thus initiating a coupling reaction to form a colored product. The so-called Gibbs Reaction is typical of such a reaction sequence, in which a 2,5-cyclohexadiene-1-one-2,6-dihalo-4-haloimine (I) couples with a phenol (II) to form a colored reaction product (III).

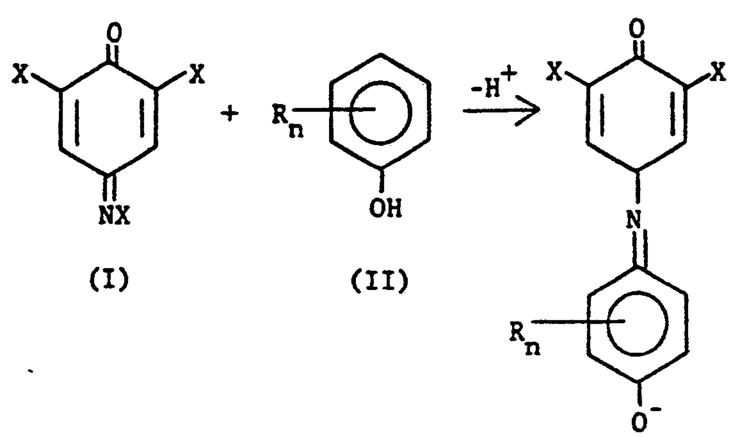


(I) (II) (III)

In this reaction sequence R, same or different, can be any 2,3,5 and/or 6-position substituent(s) which will not hinder the overall reaction sequence. Thus each R is lower or intermediate alkyl or aryl, or one R can form a fused ring system at either the 2,3- or 5,6-positions. X is a halogen or pseudohalogen, and n is 0—4. This kind of reporter substance is utilized by incorporating compounds having the structures (I) and (II) with the hydrophobic vehicle.

Still another utilization of the Gibbs chemistry involves compounds having a structure such as (III) in its nonionized form. The formation of the ion/ionophore complex results in an interaction such that reporter substance (III) yields observable color in and of itself. This phenomenon can be thought of as proceeding in accordance with the following reaction sequence and resonance structures:

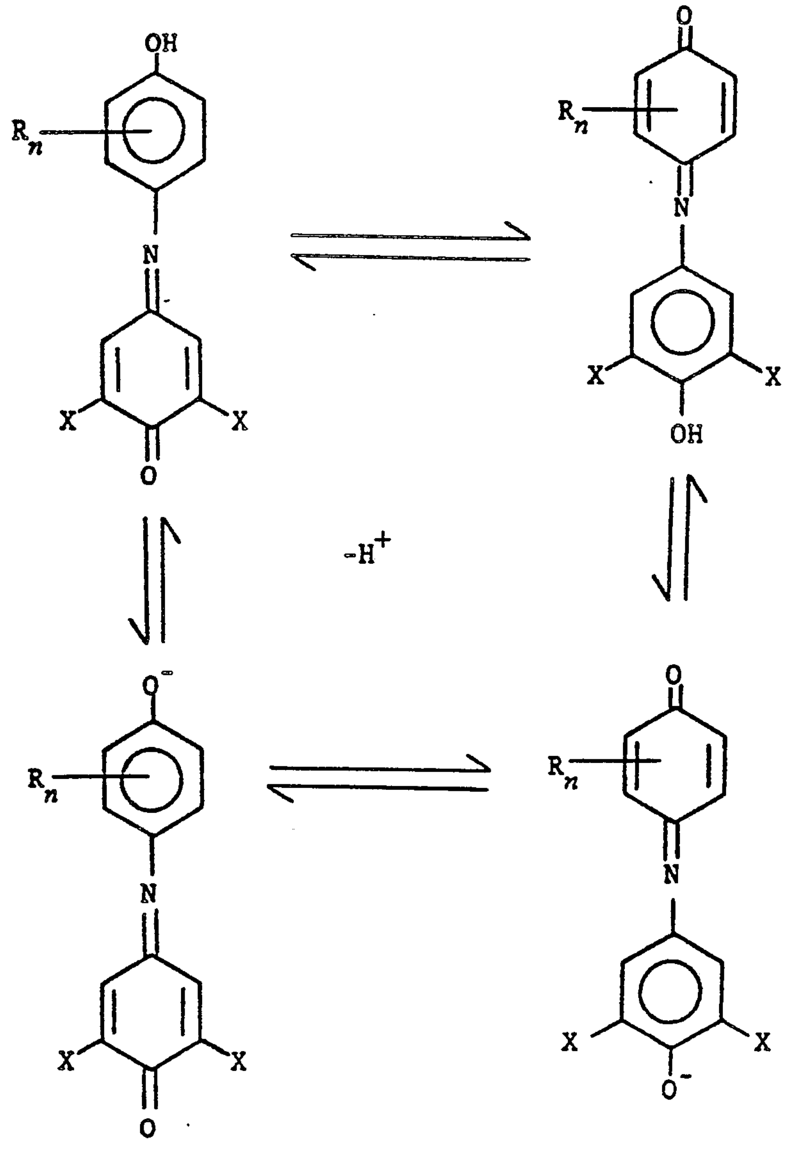


in which each R, same or different, is lower or intermediate alkyl, aryl, or a fused ring system at the 2,3- or 5,6-positions, and n is 0—4. Especially preferred is a compound having the structure

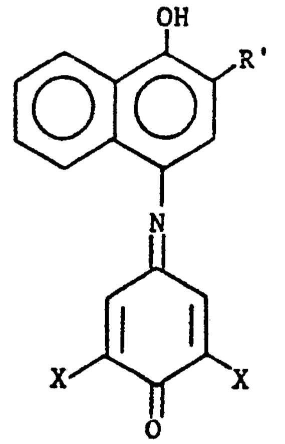

in which R′ is H or lower alkyl and X is a halogen or pseudohalogen group as defined in sections 5.6 and 5.7, respectively. The case in which R′ is methyl and X is a chlorine atom has been found especially suitable to the present invention.

Yet another preferred reporter substance is a compound having the structure

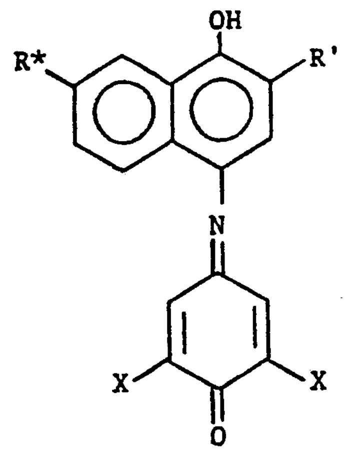

in which R* is intermediate alkyl, i.e., a group having 5—15 carbon atoms, and in which R′ is H or lower alkyl and X is a halogen or pseudohalogen (see sections 5.6 and 5.7). Compounds such as these have been found to be especially resistant to possible interference due to the presence of serum albumin in the test sample. Preferred among these types of reporter substances is 7-(*n*-decyl)-2-methyl-4-(3′,5′-dichlorophen-4′-one)indonaphthol (referred to herein as 7-decyl-MEDPIN) in which R* is *n*-decyl, X is a chloro group and R′ is methyl. More detailed information on the use and preparation of such compounds can be found in EP—A—128317 and EP—A—128318, both of which are assigned to the present assignee.

6.5 Buffering Substance

The buffering substance can be any buffer, or combination of buffers, capable of providing a pH in the range of from about 5 to 10. The buffer is chosen to facilitate the coaction of the ionophore/ion complex with the reporter substance, which coaction leads to a detectable response.

Suitable buffers include *bis*[2-hydroxyethyl]imino-*tris*[hydroxymethyl]methane; 1,3-*bis*[*tris*-(hydroxymethyl)methylamino]propane, N,N-*bis*-(2-hydroxyethyl)glycine, *tris*(hydroxymethyl)aminomethane, N-[2-acetamido]-2-iminodiacetic acid; N-2-hydroxyethylpiperazine-N′,3-propanesulfonic acid; 3[N-*tris*(hydroxymethyl)methylamino-2-hydroxypropanesulfonic acid; tetramethylammonium borate; and tetramethylammonium phosphate.

The preferred pH range depends on the reporter substance; therefore the choice of the buffer is determined by the reporter substance used and to some extent by the desired detectable response. For example when 7-decyl MEDPIN is used as the reporter the preferred pH range is from 6 to 8.5. However, when a reporter substance having a higher pKa for the dissociable proton is used, a higher pH range will be preferred; similarly when a reporter having a lower pKa for the dissociable proton is used, a lower pH range will be preferred. When the detectable response is a color change, the buffer can influence the degree of such detectable response, and a particular buffer can be chosen for color intensity optimization. For example the useful color range for the reporter, 7-decyl MEDPIN, occurs from about pH 6 to 8.5 where the color change is from orange to blue. A higher pH, pH 8.5—10, gives shades of dark blue which are difficult to distinguish visually, and a lower pH, pH 5—6, gives shades of pale yellow, also difficult to distinguish visually. Both pH extremes could be used with instrumental analysis, although the best instrumental

precision occurs at the pH range of from about 6 to 8.5. Determination of a suitable pH is a routine laboratory experiment given the present disclosure.

6.6 The Hydrophobic Polymer

Useful hydrophobic polymers are substantially nonpolar (i.e. lacking ionizable functional groups), and are miscible with the hydrophobic vehicle. These include polyvinyl chloride, polyvinylidene fluoride, polystyrene, polycarbonate, poly(4-chlorostyrene), polyvinylacetate, vinylidene chloride/vinyl chloride copolymer, vinylidene chloride/acrylonitrile copolymer, vinyl chloride/vinylacetate copolymer, polymethyl methacrylate, vinyl chloride/vinyl acetate/vinyl alcohol terpolymer, polyethylene, polypropylene and polyurethane. Of course many other polymeric materials are suitable for use as the hydrophobic polymer, and the identification of such materials is well within the skill of the art, given the present disclosure. Inclusion of a hydrophobic polymer in the hydrophobic composition increases the precision of the assay results so that the coefficient of variation is no more than 2 to 4 percent. In addition, when a hydrophobic polymer is included in the hydrophobic composition the visual response can be correlated with ion concentration semiquantitatively.

6.7 Interferent Removal system

Body fluids normally contain many cations, such as sodium ion ($Na^+$), potassium ion ($K^+$), calcium ion ($Ca^{++}$) and magnesium ion ($Mg^{++}$). Although the ionophore will usually be chosen for its selectivity for the desired ion analyte, in some cases the presence of other cations could interfere with the coaction of the ionophore with the desired ion analyte. For example, Sodium Ionophore I will bind sodium ion in preference to calcium ion in a ratio of approximately 4 to 1. In samples where the ratio of sodium ion to calcium ion is less than 4 to 1, it may be necessary to prevent the interaction of calcium ion with the ionophore to ensure the proper relationship between sodium ion concentration and the detectable response. An interferent removal system can be provided to obviate this problem.

An interferent removal system can be incorporated into the carrier matrix directly or with the buffering substance. In a preferred embodiment, the removal system is designed to interact with an interfering cation so as to keep it in the aqueous phase or otherwise prevent cation interaction with the ionophore in the hydrophobic phase. For example, ethylenediamine tetraacetic acid (EDTA) and ethylene glycol *bis*(aminoethyl)tetraacetic acid are water soluble compounds which form complexes with divalent cations, such as calcium ion. If EDTA is incorporated with a test means for the determination of sodium ion, on contact with an aqueous sample containing sodium and calcium ions, EDTA will preferentially bind $Ca^{++}$. The bound calcium ion will not substantially interfere with the formation of the ionophore/sodium ion complex. In addition, ionophores can be used to remove interfering cations if they are specific for the interfering ion and are water soluble or are modified chemically to increase their water solubility without decreasing their ability to interact with the interferent. For example, Sodium Ionophore III can be modified by the addition of solubilizing groups, such as ($-SO_3H$) groups, to the benzene rings to increase its water solubility without decreasing its ability to interact with sodium ion. Other compounds such as uramildiacetic acid and *trans*-1,2-diaminocyclohexane-N,N,N',N'-tetraacetic acid, can also be used advantageously.

6.8 The Wetting Substance

For some uses, the operation of the test means (or device) will be enhanced by the addition of a wetting substance. For example, when a sample is pipetted onto the test means it can form a liquid bead on the surface of the device. Any substance which will reduce the surface tension of the aqueous sample allowing the sample to flow into the interstices of the test matrix can act as a wetting substance. Of course the substance must be chosen so that it does not interfere with the formation of the ionophore/ion complex or with the coaction of the ionophore/ion complex and the reporter substance to produce a detectable response.

In some cases a wetting substance need not be added. For example, when Sodium Ionophore III is used, no additional wetting substance is necessary. When a wetting substance is to be added, the substance is preferentially incorporated either with the buffering substance or separately. Suitable wetting substances include agarose and detergents such as polyethylene-glycol-*p*-isooctylphenyl ether; 2-O-acetoxy-3-(perfluoroalkyl)-N-carboxymethyl-N,N-dimethylpropylamine; polyethylene glycol-1-(2-perfluoroalkyl)ethyl ether; N-decyl-N,N-dimethyl-3-ammonio-1-propanesulfonate: N-perfluoroalkyl-N-carboxyethyl-N,N-dimethylamine and polyoxyethylene ethers of fatty alcohols, in particular Brij® 35SP (obtained from ICI United States, Inc. Wilmington, Del.). Other detergents can be used provided they do not interfere with the production of a detectable response to the designated ion-analyte.

7. Concentration ranges of test means components

The concentrations of the test means components are not critical to the invention provided that the concentrations of the ionophore, hydrophobic vehicle, reporter substance and the buffering substance are sufficient to produce the desired detectable response. In some cases the production of the desired detectable response or of the desired precision may require the addition of a hydrophobic polymer.

However, for qualitative results neither the concentration of the ionophore nor the concentration of the reporter substance is tied to the concentration range of the ion-analyte to be determined.

Determination of optimum concentrations is within the ability of one skilled in the art, given the present disclosure. However, the following guidelines are provided. It is preferable that the ionophore be present in molar excess over the reporter substance (i.e., greater than 1:1 molar ratio, ionophore:reporter substance). Working concentrations of the ionophore can range from 2 g/l to saturation. The hydrophobic polymer, if added at all, is usually in concentrations ranging from 0 to 50 g/l. The interferent removal system and wetting substance, if added at all, are commonly only 0 to 30 g/l.

The working and preferred ranges for instrumental and visual ion-responsive test devices are given below:

| Instrumental | Working | Preferred |
|---|---|---|
| Hydrophobic Mixture (concentration given in grams per L of organic solvent) | | |
| Ionophore | 2—200 g/l | 50—100 g/l |
| Reporter substance | 1—50 g/l | 5—30 g/l |
| Hydrophobic vehicle | .01—.3 g/l | .02—.05 g/l |
| Hydrophobic Polymer | 0—50 g/l | 10—30 g/l |
| Wetting substance | 0—30 g/l | 0—20 g/l |
| Buffer Mixture (concentrations given relate grams to concentration per L of water or water miscible solvent) | | |
| Buffer | 0.1—1 M | 0.1—0.5 M |
| Interferent removal system | 0—30 g/l | 10—20 g/l |
| Wetting substance | 0—30 g/l | 10—20 g/l |
| Visual | | |
| Hydrophobic Mixture (concentration given in weight per final volume hydrophobic solution) | | |
| Ionophore | 2—50 g/l | 5—15 g/l |
| Reporter substance | .5—5 g/l | 1.5—4 g/l |
| Hydrophobic vehicle | 20—200 g/l | 80—150 g/l |
| Hydrophobic polymer | 10—50 g/l | 10—20 g/l |
| Buffer Mixture (concentration in final volume buffer solution) | | |
| Buffer | 0.1—1.0 M | 0.3—0.7 M |
| Interferent removal system | 0—30 g/l | 0—20 g/l |
| Wetting substance | 0—30 g/l | 0—20 g/l |

8. Test device

The test means prepared as described in Section 6 can be mounted at one end of an elongated support member, the other end of the support serving as a handle, thus forming a test device. Such a test device can be held at the handle end, while the other end bearing the test means is contacted with the test sample.

Useful materials for the support member include films of a myriad of plastics or polymers. Examples include such polymeric materials as cellulose acetate, polyethylene terephthalate, polycarbonates and

polystyrene. The support can be opaque or it can transmit light or other energy. When the detectable response is fluorescence or when a coating is placed over the upper surface of the test device to allow the sample to be wiped off, the test device can be read through the support material. In that case useful supports include transparent materials capable of transmitting electromagnetic radiation of a wavelength in the range of about 200 nanometers (nm) to 900 nm. The support need not, of course, transmit over the entire 200—900 nm region, although for fluorometric detection of analytical results it is desirable that the support be transparent over a band wider than, or at least equal to, the absorption and emission spectra of the fluorescent materials used for detection. It may also be desirable to have a support that transmits one or more narrow wavelength bands and is opaque to adjacent wavelength bands. This could be accomplished, for example, by impregnating or coating the support with one or more colorants having suitable absorption characteristics.

To prepare a test device of the present invention, a small rectangle of the test means, i.e., a doubly dried porous carrier matrix doubly incorporated with homogeneous hydrophobic composition containing an ionophore, a hydrophobic vehicle and a reporter substance, and with a buffering substance and possibly with other ingredients, is affixed to an elongated support member having an upper substantially flat face, such as an oblong piece of polystyrene film. The test means piece is affixed to the upper flat face at one end, leaving the other end of the polystyrene to serve as a convenient handle.

The test means can be affixed by any means compatible with the intended use. One method is to use a double faced adhesive tape between the test means rectangle and the support member. One such tape, known as Double Stick, is available from 3M Company, St. Paul, Minnesota.

9. Use of the invention

The test means and device of the present invention can be adapted for use in carrying out a wide variety of chemical analyses, not only in the field of clinical chemistry, but in chemical research and chemical process control laboratories. They are well suited for use in clinical testing of body fluids, such as blood, blood serum, cerebrospinal fluid and urine, since in this work a large number of repetitive tests are frequently conducted, and test results are often needed a very short time after the sample is taken. In the field of blood analysis, for example, the invention can be adapted for use in carrying out quantitative analysis for many of the ionic blood components of clinical interest.

The test means (and test device) is used by contacting it with the test sample for a sufficient period of time. In the case of urine testing merely dipping the test means (or device) into the sample is sufficient. Although it is usually unnecessary to remove excess sample, in some cases, such as whole blood samples, it is desirable to remove any excess by wiping or blotting.

If the ion under analysis is present in the test sample, the complex of ionophore and ion will interact with the reporter substance, and a detectable response will appear. Where the reporter substance is a dissociable substance producing a colored counterion of the analyte, an observable color will form in the test means which can be instrumentally monitored from either side of the device when a transparent support member is used. Where the reporter substance is a fluorophore such as fluorescein or its derivatives, a fluorescence spectrophotometer can be utilized to measure the detectable response formed in the test means (here, the appearance of or change in fluorescence). Other techniques useful in observing a detectable response include reflectance spectrophotometry, absorption spectrophotometry and light transmission measurements.

Various calibration techniques are applicable as a control for the analysis. For example, a sample of analyte standard solution can be applied to a separate test means as a comparison or to permit the use of differential measurements in the analysis. Test means (or devices) prepared with a hydrophobic composition including a hydrophobic polymer can be used for semi-quantitative visual determinations when an appropriate color chart is supplied.

10. Procedure

10.1 Preparation, General

*Test Means Preparation*

Test means responsive to a particular ion designated as the analyte were prepared by the following method:

a) forming a homogeneous first mixture of the hydrophobic composition containing at least an ionophore specific for a designated ion, a hydrophobic vehicle, a reporter substance and an organic solvent;

b) forming a homogeneous second mixture of a buffering substance capable of providing a pH in the range of from about 5 to 10 and water or a water-miscible solvent or mixtures thereof;

c) incorporating one of the first or the second mixtures with a porous carrier matrix;

d) drying the incorporated matrix;

e) incorporating the other of the first or second mixtures with the porous carrier matrix; and

f) drying the doubly incorporated carrier matrix.

Unless otherwise stated, the first mixture is a homogeneous hydrophobic solution prepared by dissolving the required components in an organic solvent. Suitable organic solvents include ketones and ethers, such as tetrahydrofuran, acetone and cyclohexanone, among others. Choice of a suitable solvent

would be routine for one skilled in the art given the present disclosure. Although a working formulation can be obtained with only the ionophore, the hydrophobic vehicle and the reporter substance; generally the mixture additionally includes a hydrophobic polymer.

The second mixture is commonly an aqueous solution containing a suitable buffer, prepared by dissolving the buffer in distilled water and titrating to the desired pH. In the following examples, the buffer solution is defined in terms of its final concentration. Whatman® type 31 ET filter paper was used as the porous carrier matrix. Each solution is incorporated into the porous carrier matrix separately. The homogeneous hydrophobic solution was incorporated with the paper first by immersing the paper in the solution. The paper was then dried in an air oven. The second incorporation was accomplished by immersing the dried paper in the aqueous buffer solution; the doubly incorporated paper was again dried in an air oven.

*Test Device Preparation*

Test devices, unless otherwise stated, were prepared by affixing a piece of double immersed and dried paper, cut from the resulting test means, to one end of a 0.5 × 10 centimeter (cm) polystyrene film strip with a double-sided adhesive tape (Double Stick from 3M Co., St. Paul, Minn.). The test device thus formed is suitable for use with the Ames SERALYZER® photometer, or for visual reading with an appropriate color chart.

10.2 Preparation, Variations

Any variation from the general procedure outlined above will be specifically noted in the examples.

In some cases the buffer solution was prepared with a water-miscible solvent, such as lower alcohols or acetone, or mixtures of water and a water-miscible solvent. For example, the buffering substance can be dissolved in distilled water, titrated to the desired pH and a mixture of water and a lower alcohol, such as methanol or ethanol, added to produce the final volume. In those cases, the buffer solution is defined in terms of its final concentration in the water/alcohol solution. When a water-miscible solvent alone is used, the buffering substance is dissolved in the solvent, titrated to a suitable pH with phosphoric acid and brought to final volume with solvent.

Normally any wetting substance and/or interferent removal system used was included in the buffer solution and incorporated into the test means in the second impregnation.

10.3 Results

A test means (or device) was tested for its response to a designated ion-analyte by contacting the device with an aqueous sample and observing any detectable response. Contact was made either by dipping the device into a sample or by pipetting a sample onto the device. Samples were either contrived aqueous samples, urine or serum samples. When body fluids samples were used, flame photometry was employed to determine the concentration of designated ion-analyte in the sample. Reflectance measurements taken a predetermined amount of time after contact were related to the concentration of the ion-analyte.

When the devices were formulated for quantitative instrumental reading, reflectance was measured on an Ames SERALYZER® reflectance photometer (Miles Laboratories, Inc., Elkhart, IN) at a wavelength between 640—700 nanometers (nm). These reflectance measurements were evaluated with a simplified form of the well known Kubelka-Munk equation [See Gustav Kortüm, "Reflectance Spectroscopy", pp 106—111, Springer Verlag, New York (1969)]:

$$K/S = \frac{(1-R)^2}{}$$

in which R is the fraction of reflectance from the test device, K is a constant and S is the light scattering coefficient of the particular reflecting medium. K/S is related to the concentration of the absorbing species, usually the deprotonated reporter substance. K/S (or some power of K/S depending on the equilibrium relationship of the concentration of the absorbing species and the ion-analyte) was plotted against increasing concentration of ion-analyte [ion], in millimoles per liter (mM) in the test sample. Least squares linear regression analysis is used to obtain the best straight line fit to the data. Where K/S is used, the straight line is represented by the equation

$$K/S = \text{Slope [ion]} + \text{intercept}$$

where [ion] is the concentration of the ion analyte in the aqueous sample; K/S is defined as above; "slope" refers to the slope of the least squares plot; and "intercept" refers to the intercept of the least squares plot. A regression coefficient, r, is calculated and indicates the scatter of the data off the straight line. Data fitting a perfect straight line plot would have a regression coefficient of one. When some power of K/S is related to the concentration of the ion analyte due to the equilibrium relationship of the ion with the absorbing species, that power of K/S will be used to define the equation, i.e., $(K/S)^2$ = slope [ion] + intercept. In any case the equation represents the response of the test device to increasing ion concentration and is often called the "dose response" curve.

A large slope indicates the device is highly sensitive since a small change in the concentration of the ion-analyte in the sample will produce a large change in response (i.e., color). On the other hand, the intercept (the data point where the ion-analyte concentration is zero) should be as small as possible as that point indicates the background (color) which is unrelated to the ion-analyte concentration.

Devices formulated with a hydrophobic polymer respond semiquantitatively to ion concentration. The ion concentration can be determined visually when an appropriate color chart is supplied. These devices were evaluated using reflectance measurements taken on a Macbeth® Colorimeter, Series 1500 (Kollmorgen Corp., Newburgh, N.Y.) at wavelengths from 400—700 nm.

These reflectance data were used to calculate the ΔE between the two samples. ΔE is a measure of the total color difference between two samples in three-dimensional color space. ΔE is calculated with the following equation: [See D. B. Judd and G. Wyszecki, "Color in Business, Science and Industry", John Wiley and Sons, New York (1975)]:

$$\Delta E = [(\Delta L^*)^2 + (\Delta a^*)^2 + (\Delta b^*)^2]^{1/2}$$

where $\Delta L^*$ is a measure of the difference in lightness between the two samples, and varies from 0 for absolute black to 100 for a perfect white; $\Delta a^*$ is a measure of the difference in redness-greenness between two samples; and $\Delta b^*$ is a measure of the difference in yellowness-blueness. The $L^*$, $a^*$, $b^*$ are calculated from the reflectance at wavelengths from 400—700 nm. ΔE between a negative sample (one which does not contain the ion-analyte) and a positive sample (one which does contain the ion analyte) will increase with increasing ion concentration. For visual testing the difference between concentration levels defined on a color chart should be as great as possible to allow the human eye to distinguish between the colors produced. Two colors are usually perceived as different by the human eye if the ΔE between them is at least three color difference units (i.e., ΔE between concentration levels should be 3 or greater). The ability of the human eye to distinguish color difference will vary to some degree depending on what region of color space is involved (i.e. changes from gold to light tan and variations in grey shades may have the same ΔE and yet the former could be distinguished more easily than the latter). Of course, such a difference is not necessary if instrumental reading is to be used.

Abbreviations used in the examples are as follows:

Square brackets, [ ], are used to designate ion concentration in millimoles per liter (mM) in the linear regression equations. All percent concentrations are given in weight per deciliter unless otherwise indicated.

| | |
|---|---|
| Temperature: | |
| °C | degrees Centigrade |
| Length: | |
| cm | centimeters |
| Weight: | |
| g | gram |
| mg | milligram |
| Volume: | |
| dl | deciliter |
| ml | milliliter |
| µl | microliter |
| l | liter |
| Concentration: | |
| mM | millimolar (millimoles per liter) |
| M | molar (moles per liter) |
| % w/v | percent weight per deciliter |
| % v/v | percent volume per deciliter |

Ions:

| | |
|---|---|
| $Na^+$ | sodium ion |
| $K^+$ | potassium ion |
| $Li^+$ | lithium ion |
| $Ca^{++}$ | calcium ion |

Abbreviations for chemical components used are given below. The ionophore designations were assigned by the present inventors for convenience only. The name is usually based on the principle ion the ionophore was used to determine. However, the ionophores commonly respond, to varying degrees, to other ions. (Structures of preferred ionophores are given in Section 6.3.4)

| | |
|---|---|
| Ionophores | |
| Sodium Ionophore I | 1,1,1-*tris*(1′-(2′-oxa-4′-oxo-5′-aza-5′-methyl)dodecanyl]propane |
| Sodium Ionophore II | N,N′-dibenzyl-N,N′-diphenyl-1,2-phenylenedioxydiacetamide |
| Sodium Ionophore III | 6,7,9,10,18,19-hexahydro-17-*n*-butyl-dibenzo[b,k][1,4,7,10,13]pentaoxacyclohexadecine-18-yl-oxyacetic acid |
| Potassium Ionophore I | 2,3-naphtho-1,4,7,10,13-pentaoxacyclopentadeca-2-ene |
| Lithium Ionophore I | N,N′-diheptyl-N,N′-5,5-tetramethyl-3,7-dioxanonane diamide |
| Lithium Ionophore II | N,N′-diheptyl-5,5-dimethyl-N,N′-di-(3-oxapentyl)-3,7-dioxanonane diamide |
| Calcium Ionophore | diethyl-N,N′-[(4R,5R)-4,5-dimethyl-1,8-dioxo-3,6-dioxaoctamethylene]*bis*(12-methylaminododecanoate) |
| Hydrophobic Vehicles | |
| NPOE | 2-nitrophenyl octyl ether |
| NPBE | 2-nitrophenyl butyl ether |
| CDA | *cis*-N,N,N′,N′-tetraisobutyl-1,2,-cyclohexane dicarboxamide |
| Reporter Substance | |
| 7-decyl MEDPIN | 7-(*n*-decyl)-2-methyl-4-(3′,5′-dichlorophen-4′-one)-indonaphthol |
| Buffering Substance | |
| Bis-Tris | *bis*[2-hydroxyethyl]imino-*tris*[hydroxymethyl]methane |
| Bis-Tris propane | 1,3-*bis*[*tris*(hydroxymethyl)methylamino]propane |
| Tris | *tris*(hydroxymethyl)aminomethane |
| ADA | N-[2-acetamido]-2-iminodiacetic acid |
| HEPPS | N-2-hydroxyethylpiperazine-N′,3-propanesulfonic acid |
| Bicine | N,N-*bis*[2-hydroxyethyl]glycine |
| TMA borate | tetramethylammonium borate |
| TMA phosphate | tetramethylammonium phosphate |
| TAPSO | 3[N-*tris*(hydroxy methyl)methylamino]-2-hydroxypropane sulfonic acid (obtained from P. L. Biochemicals, Inc., Milwaukee, WI) |

Hydrophobic Polymer (All obtained from Aldrich Chemical Co., Milwaukee, WI unless otherwise noted)

| | |
|---|---|
| PVC | polyvinyl chloride |
| (low MW) | low molecular weight |
| (very high MW) | very high molecular weight |
| VdC/VC | vinylidene chloride/vinyl chloride copolymer (Scientific Polymer Products, Ontario, N.Y.) |
| VdC/AN | vinylidene chloride-acrylonitrile copolymer (Scientific Polymer Products, Inc., Ontario, N.Y.) |
| PC—I | polycarbonate (molecular weight 20,000 to 25,000) |
| PC—II | polycarbonate (molecular weight 33,800) |
| PC—III | polycarbonate (molecular weight 38,100) |

Miscellaneous

| | |
|---|---|
| THF | tetrahydrofuran |
| EDTA | ethylenediamine tetraacetic acid |
| EGTA | ethylene glycol-*bis*(aminoethyl)tetraacetic acid (G. Frederick Smith Chemical Co., Columbus, Ohio) |
| Triton® X—100 | polyethylene glycol-*p*-isooctylphenyl ether (Sigma Chemical Co., St. Louis, MO) |
| Zonyl® FSK | 2-O-acetoxy-3-(perfluoroalkyl)-N-carboxymethyl-N,N-dimethylpropylamine (Dupont Chemical Co., Wilmington, Del) |
| Zonyl® FSN | polyethylene glycol-1-(2-perfluoroalkyl)ethyl ether (Dupont Chemical Co., Wilmington, Del) |
| Zonyl® FSB | N-perfluoroalkyl-N-carboxyethyl-N,N-dimethylamine (Dupont Chemical Co., Wilmington, Del) |
| Zwittergent® 3-10 | *n*-decyl-N,N-dimethyl-3-ammonio-1-propanesulfonate, (Calbiochem-Behring, San Diego, CA) |
| Brij® 35SP | polyoxyethylene ethers of fatty alcohols (ICI United States, Inc. Wilmington, Del) |

## 11. Examples

The present invention will now be illustrated, but is not intended to be limited, by the following examples.

### Example 11.1

Sodium Ion: Sodium Ligand I

Test means (and devices) responsive to the concentration of sodium ion in aqueous samples were prepared as described in the general procedure.

The hydrophobic solution was composed of:

| | |
|---|---|
| Sodium Ligand I | 6 mg |
| NPOE | 0.3 ml |
| PVC (low MW, 10% in cyclohexanone) | 0.6 ml |
| 7-decyl MEDPIN | 5.4 mg |
| cyclohexanone | 1.35 ml |

After the first impregnation the paper was dried at 50°C for 40 minutes. The buffer solution contained 0.3 M Bis-Tris, pH 7.5. The second drying was done at 50°C for 30 minutes. A 0.6 × 0.6 cm square of the doubly impregnated and dried paper was used to form a test device. This formulation was tested to determine if visually discernable color levels would develop when the device was dipped into an aqueous sample containing from 0 to 200 mM sodium ion. The results were positive, with the color level developed corresponding semiquantitatively to the sodium ion concentration present.

Example 11.2

Sodium Ion: A Preferred Embodiment

A preferred formulation of test means (and devices) responsive to the concentration of sodium ion in aqueous samples was prepared as described in the general procedure.

The hydrophobic solution contained:

| | |
|---|---|
| Sodium Ligand I | 300 mg |
| NPOE | 4.5 ml |
| PVC (high MW, 5% in THF) | 15 ml |
| 7-decyl-MEDPIN | 81 mg |
| THF | 12.75 ml |

The buffer solution contained 0.45 M Bis-Tris, pH 7.5, and 0.05% polyvinylmethyl ether (Gantrez® M—154 from GAF Corp. New York, N.Y.). Both drying steps were done at 65°C for 7 minutes. A 5,08 × 10,16 mm (1/5 × 2/5 inch) piece of the doubly dried and impregnated paper was used to form a test device.

Testing was done with urine samples containing 0, 30, 70, 120 or 200 mM sodium ion. Devices dipped in different samples could be distinguished visually. Reflectance spectra were taken 30 seconds after the devices were dipped. ΔE calculation gave the following results:

| $[Na^+]$, mM | ΔE between levels |
|---|---|
| 30 | 5.97 |
| 70 | 6.65 |
| 120 | 4.16 |
| 200 | |

Since the difference in ΔE from one concentration level to another was well over 3 units; these concentration levels could easily be visually distinguished.

Example 11.3

Sodium Ion: Alternate Ionophore

An alternate ionophore for the determination of sodium ion concentration in aqueous samples was tested using the formulation of the present invention.

Test means (and devices) were prepared as described in the general procedure, using an alternate ionophore, Sodium Ionophore II. The hydrophobic solution contained:

| | |
|---|---|
| Sodium Ionophore II | 50 mg |
| NPOE | 0.75 ml |
| PVC (low MW, 10% in cyclohexanone) | 1 ml |
| 7-decyl MEDPIN | 14 mg |
| cyclohexanone | 3.3 ml |

After the first impregnation the paper was dried at 90°C for 20 minutes. The buffer solution was 0.3 M Bis-Tris, pH 7.5. The second drying was done at 75°C for 20 minutes. A 5,08 × 10,16 mm (1/5 × 2/5 inch) piece of doubly impregnated and dried paper was used to form a test device.

When test devices were dipped in urine samples containing 14, 34, 68, 119 mM sodium ion, visually discernable color developed. Reflectance spectra were taken 2 minutes after the devices were dipped in the urine samples. ΔE calculations gave the following results:

| [Na⁺], mM | ΔE between levels |
|---|---|
| 14 | 8.68 |
| 34 | 11.03 |
| 68 | 1.37 |
| 119 | |

The results indicate that the human eye could distinguish between the color developed after contact with the three lower concentrations, but would not be able to distinguish the color difference between the 68 mM and 119 mM concentrations.

### Example 11.4

Sodium Ion: Order of Incorporation Changed

Test means (and devices) responsive to the concentration of sodium ion in aqueous samples were prepared. The general procedure was altered by impregnating the Whatman paper first with the buffer solution prepared in methanol and second with the hydrophobic solution. The buffer solution contained 0.3 M Bis-Tris (in methanol), pH 7.5. After impregnating with the buffer, the paper was dried at 90°C for 20 minutes.

The hydrophobic solution contained:

| | |
|---|---|
| Sodium Ligand I | 40 mg |
| PVC (low MW, 10% in cyclohexanone) | 4.0 ml |
| NPOE | 3.08 ml |
| 7-decyl MEDPIN | 56 mg |
| cyclohexanone | 12.92 ml |

The second drying was done at 75°C for 20 minutes. A 5,08 × 10,16 mm (1/5 × 2/5 inch) piece of the doubly impregnated and dried paper was used to form a test device.

When the devices formulated by reversing the order of impregnation were dipped in urine samples, color was visually discernable and corresponded semiquantitatively to sodium ion concentration, [$Na^+$], as measured by flame photometry. Reflectance spectra were taken 30 seconds after the devices were dipped in urine samples.

ΔE calculations gave the following results:

| [Na⁺], mM | ΔE between levels |
|---|---|
| 21 | 2.59 |
| 51 | 12.52 |
| 101 | |

Color levels obtained for the lower concentration levels were slightly closer than 3 color units. However at low concentrations of sodium ion, this device formulation changes from gold to light tan which can be more easily distinguished visually than the same degree (ΔE between levels) of color change at higher sodium ion concentrations. For example at 100 mM sodium ion and greater this formulation exhibits varying shades of grey which are more difficult to distinguish visually.

### Example 11.5

Potassium Ion: A Preferred Embodiment

Test means (and devices) responsive to the concentration of potassium ion in an aqueous sample were prepared according to the general procedure.

The hydrophobic solution contained:

| | | |
|---|---|---|
| Potassium Ionophore I | 50 | mg |
| NPOE | 0.6 | ml |
| VdC/AN | 300 | mg |
| PC—II | 110 | mg |
| 7-decyl MEDPIN | 19 | mg |
| THF | 25 | ml |

The buffer solution, pH 6.26, contained 500 mM ADA, 667 mM Tris and 0.3% of a surfactant, Triton® X—100, in distilled water. Each drying step was done at 60°C for 10 minutes. A 0.2 × 0.4 cm piece of the doubly impregnated and dried paper was used to prepare the test device.

Aqueous potassium chloride solutions were used as samples. For each concentration of potassium ion, device reactivities were measured in triplicate by reflectance and the mean K/S value calculated.

| $[K^+]$, mM | Mean K/S |
|---|---|
| 2 | 0.4750 |
| 3 | 0.6114 |
| 4 | 0.7119 |
| 5 | 0.8555 |
| 6 | 0.9592 |

Least squares analysis of the data (Mean K/S versus mM potassium ion concentration) gave the following linear relationship:

$$K/S = 0.121\ [K^+] + 0.238;\ r = 0.9986$$

The test device gave a positive response to the presence of potassium ion with good correlation between K/S and potassium ion concentration.

Example 11.6

Potassium Ion: Ionophore Variation

A series of test means (and devices) were prepared according to the general procedure, utilizing different potassium selective ionophores.

The hydrophobic solution contained:

| | | |
|---|---|---|
| Ionophore (see below) | | |
| NPOE | 2.4 | ml |
| VdC/AN | 254 | mg |
| PC—II | 254 | mg |
| 7-decyl MEDPIN | 76 | mg |
| THF | 100 | ml |

The buffer solution, pH 6.6, contained 300 mM ADA, 471 mM Tris and 0.2% of a surfactant, Triton® X—100, in distilled water. Both drying steps were done at 60°C for 10 minutes. A 0.2 × 0.4 cm piece of the doubly impregnated and dried paper was used to form test devices suitable for use with the Ames SERALYZER® photometer.

The ionophores used in the hydrophobic solution are listed below. All were obtained from Parish Chemical Co., Orem, Utah.

| Ionophore | | |
|---|---|---|
| A | Cyclohexyl-15-crown-5 | 47.6 µl |
| B | benzo-15-crown-5 | 50.6 mg |
| C | 4-acetylbenzo-15-crown-5 | 58.6 mg |
| D | 4-(1-hydroxyethyl)benzo-15-crown-5 | 58.9 mg |
| E | 4-(1-hydroxymyristyl)benzo-15-crown-5 | 90.7 mg |
| F | 4-t-butylbenzo-15-crown-5 | 61.2 mg |
| G | Potassium Ionophore I | 60.0 mg |

Aqueous potassium chloride samples were prepared containing 0, 2, 4, 6, 8 and 10 mM potassium ion. In addition, aqueous sodium chloride samples were prepared containing 0, 40, 80, 120, 160 and 200 mM sodium ion. Each formulation was tested for response to the potassium ion samples and to the sodium ion samples. Each sample was pipetted onto a test device and reflectance measurements taken. Results of the least squares regression analysis of the data (calculated K/S values versus ion concentration in mM) for the response of each formulation to sodium ion and to potassium ion are tabulated below.

| Ionophore | Dose Response | Selectivity $K^+/Na^+$ |
|---|---|---|
| A | K/S = 0.1540 + 0.02371 [$K^+$] | 62 |
| A | K/S = 0.1440 + 0.000383 [$Na^+$] | |
| B | K/S = 0.1293 + 0.006576 [$K^+$] | 41 |
| B | K/S = 0.1255 + 0.000162 [$Na^+$] | |
| C | K/S = 0.1376 + 0.00633 [$K^+$] | 73 |
| C | K/S = 0.1437 + 0.000091 [$Na^+$] | |
| D | K/S = 0.1562 + 0.00904 [$K^+$] | 60 |
| D | K/S = 0.1477 + 0.00015 [$Na^+$] | |
| E | K/S = 0.2901 + 0.1527 [$K^+$] | 160 |
| E | K/S = 0.1793 + 0.00096 [$Na^+$] | |
| F | K/S = 0.4253 + 0.1606 [$K^+$] | 179 |
| F | K/S = 0.1805 + 0.000896 [$Na^+$] | |
| G | K/S = 0.2734 + 0.1171 [$K^+$] | 276 |
| G | K/S = 0.1409 + 0.00042 [$Na^+$] | |

The results indicate the dose response of each formulation to sodium ion and to potassium ion. In each case the response to potassium ion was at least an order of magnitude greater than the response to sodium ion. Selectivity of each formulation for potassium ion over sodium ion was calculated as the ratio of the slope of the derived equation for potassium ion over the slope of the derived equation for sodium ion.

Example 11.7

Potassium Ion: Serum Samples

Test means (and devices) responsive to the concentration of potassium ion were prepared by the general procedure.

The hydrophobic solution contained:

| | |
|---|---|
| Potassium Ionophore I | 1.875 g |
| NPOE | 15 ml |
| PC—II | 3.0 g |
| VdC/AN | 6.0 g |
| 7-decyl MEDPIN | 0.75 g |
| THF | 600 ml |

The buffer solution, pH 6.6, was prepared by dissolving 42.8 g ADA, 42.8 g of Tris, and 1.5 gm of a wetting substance Zonyl® FSB in 750 ml of distilled water. Both drying steps were done at 60°C for 10 minutes. A 0.2 × 0.4 cm piece of the doubly impregnated paper was used to prepare a test device.

Devices so prepared were tested for the response to potassium ion in serum samples. The reflectance of the test devices was measured on an Ames SERALYZER® reflectance photometer between 60 and 75 seconds after contact with the serum samples. Readings were taken at 640 nanometers.

K/S, calculated as a function of reflectance measurements, was plotted against the concentration of potassium ion (mM) in each sample (as determined by flame photometry). Least squares analysis of the data gave the relationship:

$$K/S = 0.1198\ [K^+] + 0.608$$

indicating the formulation gave a good response to serum potassium concentration.

Example 11.8

Potassium Ion: Polymer Variation

Two series of test means (and devices) were prepared by the general procedure with different hydrophobic polymers.

The hydrophobic solution contained:

| | |
|---|---|
| Potassium Ionophore I | 188 mg |
| NPOE | 0.60 ml |
| hydrophobic polymer | 0.90 g |
| 7-decyl MEDPIN | 75 mg |
| THF | 60 ml |

The buffer solution, pH 6.6, was prepared containing 300 mM ADA, 470 mM Tris and 0.2% of a wetting substance, Triton® X—100, in distilled water. Both drying steps were done at 60°C for 10 minutes. A 0.2 × 0.4 cm piece of the doubly impregnated and dried paper was used to prepare a device.

Test means, set A, were formulated using polycarbonate (PC—II) as the hydrophobic polymer. A second set, B, was formulated using vinylidene chloride/acrylonitrile copolymer, (VdC/AN), as the hydrophobic polymer. The device response to potassium ion concentration was determined by contacting a device with aqueous solutions containing 2, 3, 4, 5 or 6 mM potassium chloride and measuring its reflectance. Both formulations responded to the concentration of potassium ion but the reactivity, as indicated by the slope, and the background interference, as indicated by the intercept of the dose response curve, changed. Least squares linear regression analysis for each formulation gave the following dose response equations:

A $K/S = 0.2214 + 0.0510\ [K^+];\ r = 0.9642$

B $K/S = 0.4826 + 0.1662\ [K^+];\ r = 0.9886$

Both formulations gave a positive response to potassium ion concentration with good correlation of K/S and potassium ion concentration. Formulation B exhibited a larger change in K/S with increasing potassium ion concentration than did formulation A.

Example 11.9

Potassium Ion: Variation in Hydrophobic Vehicle

Two series of test means (and devices) were prepared by the general procedure with different hydrophobic vehicles.

The hydrophobic solution contained:

| | |
|---|---|
| Potassium Ionophore I | 150 mg |
| hydrophobic vehicle | 0.60 ml |
| PC—III | 126 mg |
| 7-decyl MEDPIN | 19 mg |
| THF | 25 ml |

The buffer solution contained 300 mM ADA, 470 mM Tris and 0.2% of a wetting substance, Triton® X—100, in distilled water. Test means (and devices), series A, were prepared using 2-nitrophenyl octyl ether (NPOE) as the hydrophobic vehicle. Test means (and devices), series B, were prepared using 2-nitrophenyl butyl ether (NPBE) as the hydrophobic vehicle.

The dose response of each series of test devices to potassium ion concentration was measured using aqueous samples containing 2, 3, 4, 5 or 6 mM potassium chloride. Least squares analysis of calculated K/S values versus mM potassium concentration gave the following dose response relationships:

A $K/S = 0.2302 + 0.0747\,[K^+];\ r = 0.9995$

B $K/S = 0.3263 + 0.1150\,[K^+];\ r = 0.9962$

Both formulations exhibited good linear response to potassium ion concentration.

Example 11.10

Potassium Ion: Omission of Polymer

Test means (and devices) were prepared according to the general procedure but without the addition of hydrophobic polymer to the hydrophobic solution.

The hydrophobic solution contained:

| | |
|---|---|
| Potassium Ionophore I | 150 mg |
| NPOE | 0.6 ml |
| 7-decyl MEDPIN | 19 mg |
| THF | 25 ml |

The aqueous buffering solution, pH 6.6, contained 0.30 M ADA and 0.47 M Tris, with 0.2% of a surfactant, Triton® X—100. The doubly impregnated and dried paper was cut and used to form test devices suitable for use with the Ames SERALYZER® reflectance photometer. The devices were prepared and the response to aqueous solutions containing 2, 3, 4, 5 and 6 mM potassium chloride was measured at 640 nanometers as described in the general procedure. Least squares analysis of K/S values (calculated from the reflectance data) versus mM potassium ion concentration gave the following dose response relationship:

$$K/S = 0.1888 + 0.0435\,[K^+];\ r = 0.998$$

The formulation showed a positive response to potassium ion concentration even without the addition of a hydrophobic vehicle.

Example 11.11

Lithium Ion: Lithium Ionophore II

Test means (and devices) responsive to the concentration of lithium ion were prepared as described in the general procedure.

The hydrophobic solution contained:

| | |
|---|---|
| Lithium Ionophore II | 10 mg |
| CDA | 0.75 ml |
| PVC (low MW, 10% in cyclohexanone) | 1.0 ml |
| 7-decyl MEDPIN | 14 mg |
| cyclohexanone | 3.3 ml |

The buffering solution was 0.3 M Bis-Tris, pH 7.5. Both drying steps were done at 65° for 7 minutes. A doubly impregnated and dried paper was used to form a test device.

When the devices were dipped into aqueous samples containing from 0 to 50 mM lithium ion, visually discernible color levels developed which corresponded, semiquantitatively, to the concentration of lithium ion.

The ΔEs calculated from reflectance measurements taken 5 minutes after a device was dipped in the samples for each lithium ion concentration tested, were:

| $[Li^+]$, mM | ΔE between levels |
|---|---|
| 1 | 5.91 |
| 5 | 4.61 |
| 10 | 2.31 |
| 15 | 6.24 |
| 25 | 3.82 |
| 50 | |

Concentration levels 1, 5, 15, 25 and 50 mM could readily be distinguished visually using the formulation. While 10 mM lithium ion could be distinguished from 15 mM, visual distinction of 5 mM from 10 mM would be more difficult. Of course all the concentration levels could readily be distinguished instrumentally.

## Example 11.12

Lithium Ion: A Preferred Embodiment

Test means (and devices) were prepared using Lithium Ionophore II. The hydrophobic solution was prepared by mixing 0.04 ml of a solution containing 1.008 g CDA, 18 mg 7-decyl MEDPIN and 90 µl Lithium Ionophore II; and 14.6 ml of a solution containing 0.19 gm VdC/VC and 25 ml THF. The paper was dried and then immersed in a buffer solution containing 0.20 M bicine, adjusted to pH 8.5, and 0.5% Triton® X—100. The doubly impregnated paper was oven dried at 60°C.

The test means were evaluated by reflectance readings for response to lithium ion concentration, with aqueous samples containing from 0 to 10 mM lithium chloride. A least squares analysis of the data $[(K/S)^2$ calculated from the reflectance measurements at increasing mM lithium ion concentration] gave the following relationship:

$$(K/S)^2 = 0.3146 + 0.03691\,[Li^+];\ r = 0.988$$

The equation derived shows that the device responds to increasing lithium ion concentration with good correlation of calculated $(K/S)^2$ and lithium ion concentration.

## Example 11.13

Lithium Ion: Alternate Buffers

Test means (and devices) were prepared as described in the general procedure. The hydrophobic solution was prepared as described in Example 11.11. The buffer solution contained 0.3 M HEPPS, pH 7.5.

Evaluation on a Macbeth® colorimeter 5 minutes after devices were dipped into aqueous samples containing lithium ion gave the following results:

| $[Li^+]$, mM | ΔE between levels |
|---|---|
| 10 | 5.07 |
| 25 | 6.33 |
| 50 | |

A second set of test devices was prepared using a buffer solution containing 0.2M Tris, pH 7.5. Similar evaluation procedures on aqueous samples containing lithium ion gave the following results:

| $[Li^+]$, mM | ΔE between levels |
|---|---|
| 10 | 6 |
| 25 | 5.61 |
| 50 | |

Both formulations exhibited approximately the same color change from one sample concentration to the next, but as indicated in Section 6.5 the choice of buffer affected the color development.

Example 11.14

Lithium Ion: Removal of Calcium Ion Interference

Test means (and devices) responsive to the presence of lithium ion were prepared with the addition of ingredients intended to eliminate calcium ion interference. Serial impregnation and drying of Whatman filter paper was used to prepare the test means.

The hydrophobic solution was prepared by mixing 0.4 ml of a solution containing 90 µl Lithium Ionophore I, 1.004 g CDA and 9 mg 7-decyl MEDPIN with 14.6 ml of a solution containing 19 mg VdC/AN and 25 ml THF. The paper was immersed in the hydrophobic solution and dried. The dried paper was then immersed in a solution containing 1% agarose and 0.2% Triton® X—100 and dried. The doubly dried paper was immersed in a third solution, pH 8.50, containing the buffering substance and interferent removal system composed of 0.46 M Tris, 0.05 M $MgSO_4{\cdot}7H_2O$ (magnesium sulfate heptahydrate), 0.1% Triton® X—100 and 0.05 M EGTA. After the third impregnation the paper was dried again. A piece of the triply impregnated and dried paper was used to form test devices suitable for use with an Ames SERALYZER® reflectance photometer. Samples containing 0, 2, 4, 6, 8 or 10 mM calcium chloride were pipetted onto the devices. Reflectance measurements on reacted devices, were used to calculate $(K/S)^2$. The calculated values were then used in a least squares linear regression analysis to determine if the devices containing the calcium interferent removal system responded to the presence of calcium ion. The dose response equation obtained was:

$$(K/S)^2 = 0.1488 - 9.77 \times 10^{-5}[Ca^{++}];\ r = -0.068$$

indicating that the presence of calcium ion has no effect on devices formulated with the calcium ion interferent removal system.

Example 11.15

Lithium Ion: Ionophore as the Interferent Removal System

Test means (and devices) responsive to the presence of lithium ion are prepared as described in Example 11.12. To reduce the interference of sodium ion with the coaction of a lithium ionophore with lithium ion, a water soluble, sodium specific ionophore such as

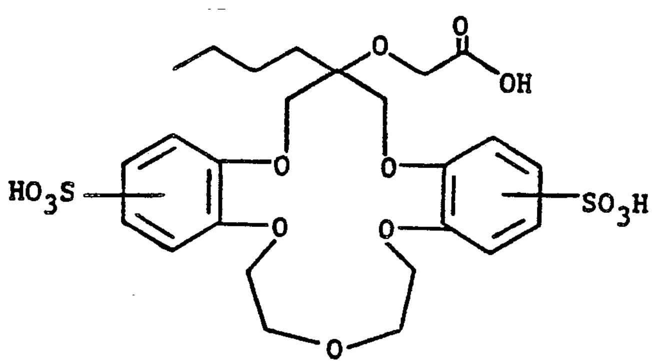

is incorporated into the paper with the buffering solution. The water soluble ionophore is produced by the chemical addition of solubilizing groups, such as $—SO_3H$, to an ion-specific ionophore in such a way that the chemical modification does not interfere with the formation of the desired ionophore/ion complex. The coaction of sodium ion with the interferent removal ionophore in the aqueous phase will retain the sodium ion in the aqueous phase and prevent the interfering ion from entering the hydrophobic phase, where it could interact with the lithium ionophore.

Other cation interferences are removed by utilizing suitably selective ionophores which can be made water soluble by chemical modification.

## Example 11.16

Calcium Ion

An early formulation of test means (and devices) was prepared as described in Example 11.3.

The hydrophobic solution contained:

| | |
|---|---|
| Calcium Ionophore | 20 mg |
| NPOE | 0.75 ml |
| PVC (low MW, 10% in cyclohexanone) | 1.0 ml |
| 7-decyl MEDPIN | 14 mg |
| cyclohexanone | 3.3 ml |

The buffering solution contained 0.3 M Bis-Tris propane, pH 7.5, in distilled water. A piece of doubly impregnated and dried paper was used to form a device as described in the general procedure.

The results of instrumental evaluation on the Macbeth® colorimeter, 30 seconds after dipping the devices in prepared aqueous solutions containing calcium ion, were:

| $[Ca^{++}]$, mM | ΔE between levels |
|---|---|
| 5 | 5.23 |
| 15 | 3.50 |
| 20 | |

The results indicate that the formulation can be used to prepare devices for visual correlation with the semiquantitative concentration of calcium ion in aqueous solution ranges.

## Example 11.17

Calcium Ion: A Preferred Embodiment

Test means (and devices) were prepared by serial impregnation and drying as described in the general procedure, except that EGTA, a strong calcium binder, was added to the buffering solution. The concentration of EGTA was adjusted so that the test device would not respond to the presence of calcium ion until the ion concentration reached 1 mM. This was expected to allow the test device to operate with higher precision over the desired concentration range.

The hydrophobic solution contained:

| | |
|---|---|
| Calcium Ionophore | 100 mg |
| VdC/VC | 282 mg |
| PC—III | 100 mg |
| NPOE | 0.625 ml |
| 7-decyl MEDPIN | 12.5 mg |
| THF | 25 ml |

The buffering solution contained 500 mM TAPSO, 75 mg Triton® X—100 and 1 ml of a solution containing 190 mg EGTA, 123 mg $MgSO_4 \cdot 7H_2O$ and 1297 mg TAPSO in a final volume of 10 ml in distilled water.

A piece of the doubly impregnated dried paper was used to form a test device. The devices were tested on aqueous calcium chloride solutions containing from 0 to 4 mM calcium ion in 0.25 mM increments and reflectance measurements were taken on an Ames SERALYZER® reflectance photometer. Due to the equilibrium relationship which indicates calcium ion concentration is proportional to $(K/S)^3$, $(K/S)^3$ was calculated for each concentration level. That data was used in least squares linear regression analysis. The dose response thus determined was:

$$(K/S)^3 = -1.711 + 1.938\,[Ca^{++}];\ r = 0.997$$

The negative intercept is due to the computed extrapolation of a dose-response curve that shows no response until the concentration of calcium ion reaches 1 mM. The results indicate the devices thus formed

show a significant response to calcium ion concentration, providing the high precision required for serum calcium determinations.

**Claims**

1. A test means for determining the presence of an ion in an aqueous test sample, the test means comprising a porous carrier matrix substantially uniformly incorporated with

a) a homogeneous hydrophobic composition containing an ionophore capable of forming a complex with the ion, a hydrophobic vehicle, and a reporter substance capable of interacting with the complex of the ionophore and ion to produce a detectable response; and

b) a buffering substance capable of providing a pH in the range of from about 5 to 10.

2. The test means of Claim 1 in which the porous carrier matrix is paper.

3. The test means of Claim 1 or 2 in which the ionophore is an uncharged podand.

4. The test means of any one of Claims 1 to 3 in which the homogeneous hydrophobic composition additionally contains a hydrophobic polymer.

5. The test means of any one of Claims 1 to 4 in which the porous carrier matrix is additionally incorporated with a wetting substance and/or or an interferent removal system.

6. The test means of any one of Claims 1 to 5 in which the reporter substance is one capable of producing the appearance of, or change in, color or fluorescence in the presence of the complex of the ionophore and ion.

7. The test means of any one of Claims 1 to 6 in which the reporter substance is a compound having the structure

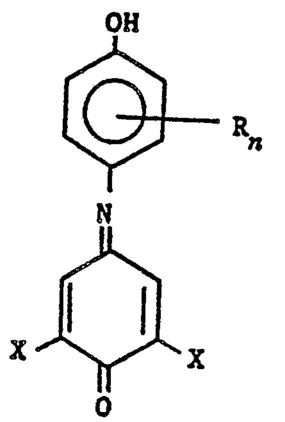

in which X is a halogen or —CN, —SCN, —OCN, —$N_3$, COZ, —COOZ, CONHZ, —$CF_3$, —$CCl_3$, —$NO_2$, —$SO_2CF_3$, —$SO_2CH_3$, —$SO_2$, —$C_6H_5$, —$SO_2C_6H_4CH_3$, —$SOC_6H_5$ or —$SOCF_3$ in which Z is alkyl or aryl; in which each R, same or different, is a 2-, 3-, 5-, and/or 6-position substituent selected from lower alkyl having 1 to 4 carbon atoms, intermediate alkyl having from 5 to 15 carbon atoms, aryl and a fused ring at the 2,3- or 5,6- positions; and N is 0 to 4; or the structure

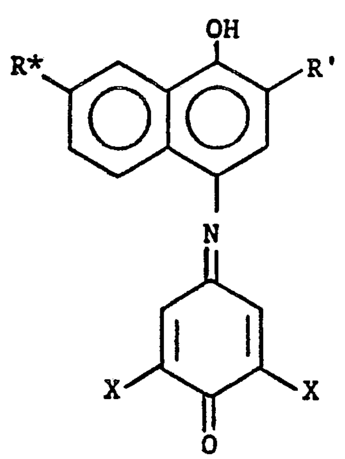

in which R′ is H or lower alkyl having 1 to 4 carbon atoms, preferably methyl, R* is H or intermediate alkyl having 5 to 15 carbon atoms, preferably n-decyl, and X is halogen or —CN, —SCN, —OCN, —$N_3$, —COZ, —COOZ, —CONHZ, —$CF_3$, —$CCl_3$, —$NO_2$, —$SO_2CF_3$, —$SO_2CH_3$, $SO_2$, —$C_6H_5$, —$SO_2C_6H_4CH_3$, —$SOC_6H_5$ or —$SOCF_3$ in which Z is alkyl or aryl.

8. A method for preparing a test means for determining the presence of an ion in an aqueous test sample, the method comprising the steps of

a) forming a homogeneous first mixture of the hydrophobic composition comprising an ionophore capable of forming a complex with the ion, a hydrophobic vehicle, a reporter substance capable of interacting with the complex of the ionophore and the ion to produce a detectable response and an organic solvent;

b) forming a homogeneous second mixture of a buffering substance capable of providing a pH in the range of from about 5 to 10 and water or a water-miscible solvent or mixtures thereof;

c) incorporating one of the first or the second mixtures with a porous carrier matrix;

d) drying the incorporated matrix;

e) incorporating the other of the first or second mixtures with the porous carrier matrix to form a doubly incorporated matrix; and

f) drying the doubly incorporated carrier matrix.

9. The method of Claim 8 in which the first mixture additionally contains a hydrophobic polymer.

10. The method of Claims 8 or 9 in which the second mixture additionally contains a wetting substance and/or an interferant removal system.

11. A process for determining the presence of an ion in an aqueous test sample, the process comprising contacting the test sample with the test means of any one of Claims 1 to 7 and observing a detectable response.

**Patentansprüche**

1. Testmittel zur Bestimmung der Gegenwart eines Ions in einer wässrigen Testprobe, wobei das Testmittel eine poröse Trägermatrix umfasst, in welche im wesentlichen gleichmässig eingebracht wurde:

a) eine homogene hydrophobe Zusammensetzung, enthalten ein Ionophor, das in der Lage ist, einen Komplex mit dem Ion zu bilden, einen hydrophoben Träger und eine Reportersubstanz, die in der Lage ist, mit dem Komplex des Ionophors und des Ions unter Ausbildung einer nachweisbaren Ansprechung zu reagieren, und

b) eine Puffersubstanz, welche einen pH-Wert im Bereich von etwa 5 bis 10 ergibt.

2. Testmittel gemäss Anspruch 1, in welchem die poröse Trägermatrix Papier ist.

3. Testmittel gemäss Anspruch 1 oder 2, in welchem das Ionophor ein ungeladener Podand ist.

4. Testmittel gemäss einem der Ansprüche 1 bis 3, in welchem die homogene, hydrophobe Zusammensetzung zusätzlich ein hydrophobes Polymer enthält.

5. Testmittel gemäss einem der Ansprüche 1 bis 4, in welchem die poröse Trägermatrix zusätzlich eine Befeuchtungssubstanz und/oder ein System zur Entfernung störender Substanzen enthält.

6. Testmittel gemäss einem der Ansprüche 1 bis 5, in welchem die Reportersubstanz in der Lage ist, in Gegenwart des Komplexes aus dem Ionophor und dem Ion das Auftreten oder die Veränderung der Farbe oder Fluoreszenz zu bewirken.

7. Testmittel gemäss einem der Ansprüche 1 bis 6, in welchem die Reportersubstanz eine Verbindung der Struktur

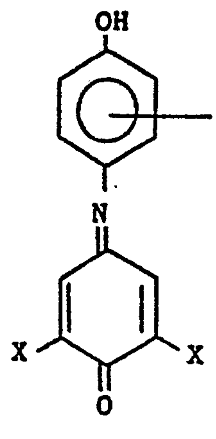

ist, in welcher X ein Halogen oder —CN, —SCN, —OCH, —$N_3$, —COZ, —COOZ, —CONHZ, —$CF_3$, —$CCl_3$, —$NO_2$, —$SO_2CF_3$, —$SO_2CH_3$, —$SO_2C_6H_5$, —$SO_2C_6H_4CH_3$, —$SOC_6H_5$ oder —$SOCF_3$ ist, wobei Z Alkyl oder Aryl bedeutet, R das gleich oder verschieden ist, ein Substituent in der 2-, 3-, 5- und/oder 6-Stellung, ausgewählt aus Niedrigalkyl mit 1 bis 4 Kohlenstoffatomen, mittlerem Alkyl mit 5 bis 15

Kohlenstoffatomen, Aryl und einem in den 2,3- oder 5,6-Stellungen ankondensierten Ring ist, und n 0 bis 4 bedeutet; oder eine Verbindung der Struktur

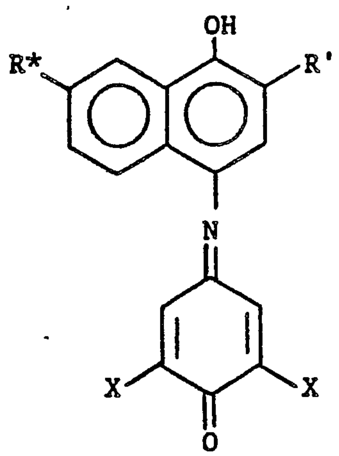

ist, worin R' H oder Niedrigalkyl mit 1 bis 4 Kohlenstoffatomen, vorzugsweise Methyl, ist, R* H oder mittleres Alkyl mit 5 bis 15 Kohlenstoffatomen, vorzugsweise n-Decyl, ist und X Halogen oder $-CN$, $-SCN$, $-OCN$, $-N_3$, $-COZ$, $-COOZ$, $-CONHZ$, $-CF_3$, $-CCl_3$, $-NO_2$, $-SO_2CF_3$, $-SO_2CH_3$, $-SO_2C_6H_5$, $-SO_2C_6H_4CH_3$, $-SOC_6H_5$ oder $-SOCF_3$ ist, worin Z Alkyl oder Aryl bedeutet.

8. Verfahren zur Herstellung eines Testmittels zur Bestimmung der Gegenwart eines Ions in einer wässrigen Testprobe, dadurch gekennzeichnet, dass es folgende Stufen umfasst;

a) Ausbilden einer homogenen ersten Mischung der hydrophoben Zusammensetzung aus einem Ionophor, das in der Lage ist, mit dem Ion einen Komplex zu bilden, einem hydrophoben Träger, einer Reportersubstanz, die in der Lage ist, mit dem Komplex aus dem Ionophor und dem Ion unter Ausbildung einer nachweisbaren Ansprechung zu reagieren, und einem organischen Lösungsmittel;

b) Ausbilden einer homogenen zweiten Mischung aus einer Puffersubstanz, die einen pH-Wert im Bereich von etwa 5 bis 10 verleiht, und Wasser oder einem wassermischbaren Lösungsmittel oder Mischungen davon;

c) Einbringen einer der ersten oder zweiten Mischungen in die poröse Trägermatrix;

d) Trocknen der so erhaltenen Matrix;

e) Einbringen der anderen der ersten oder zweiten Mischungen in die poröse Trägermatrix unter Ausbildung einer zweifach inkorporierten Matrix; und

f) Trocknen der zweifach inkorporierten Trägermatrix.

9. Verfahren gemäss Anspruch 8, bei welchem die erste Mischung zusätzlich ein hydrophobes Polymer enthält.

10. Verfahren gemäss Ansprüchen 8 oder 9, bei welchem die zweite Mischung zusätzlich eine Befeuchtungssubstanz und/oder ein System zur Entfernung störender Komponenten enthält.

11. Verfahren zur Bestimmung der Gegenwart eines Ions in einer wässrigen Testprobe, dadurch gekennzeichnet, dass man die Testprobe mit dem Testmittel gemäss einem der Ansprüche 1 bis 7 in Berührung bringt und eine nachweisbare Ansprechung beobachtet.

**Revendications**

1. Un élément d'essai pour déterminer la présence d'un ion dans un échantillon d'essai aqueux, l'élément d'essai comprenant une matrice de support poreuse, qui a été imprégnée de manière à peu près uniforme par

a) une composition hydrophobe homogène contenant un ionophore capable de former un complexe avec l'ion, un véhicule hydrophobe et une substance indicatrice capable de réagir avec le complexe de l'ionophore et de l'ion pour produire une réponse détectable; et

b) une substance tampon capable de donner un pH dans la gamme d'environ 5 à 10.

2. L'élément d'essai selon la revendication 1, dans lequel la matrice de support poreuse est du papier.

3. L'élément d'essai selon la revendication 1 ou 2, dans lequel l'ionophore est un podand non chargé.

4. L'élément d'essai selon l'une quelconque des revendications 1 à 3, dans lequel la composition hydrophobe homogène contient en outre un polymère hydrophobe.

5. L'élément d'essai selon l'une quelconque des revendications 1 à 4, dans lequel on incorpore en outre dans la matrice de support poreuse une substance mouillante et/ou un système d'élimination des ions gênants.

6. L'élément d'essai selon l'une quelconque des revendications 1 à 5, dans lequel la substance indicatrice est une substance capable de produire l'apparition ou une variation de couleur ou de fluorescence en présence du complexe de l'ionophore et de l'ion.

7. L'élément d'essai selon l'une quelconque des revendications 1 à 6, dans lequel la substance indicatrice est un composé répondant à la formule:

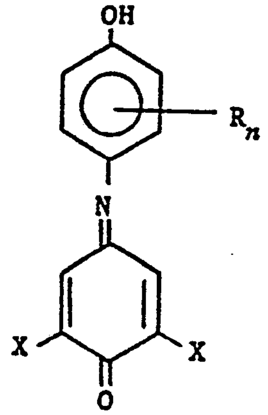

dans laquelle X est un halogène ou un groupe —CN, —SCN, —OCN, —$N_3$, —COZ, —COOZ, —CONHZ, —$CF_3$, —$CCl_3$, —$NO_2$, —$SO_2CF_3$, —$SO_2CH_3$, —$SO_2C_6H_5$, —$SO_2C_6H_4CH_3$, —$SOC_6H_5$ ou —$SOCF_3$, dans lequel Z est un groupe alkyle ou aryle; dans laquelle les R sont des substituants identiques ou différents en positions 2, 3, 5 et/ou 6 choisis parmi les groupes alkyles inférieurs ayant de 1 à 4 atomes de carbone, les groupes alkyles intermédiaires ayant de 5 à 15 atomes de carbone, les groupes aryles et un noyau accolé en positions 2,3 ou 5,6; et n est de 0 à 4; ou à la formule

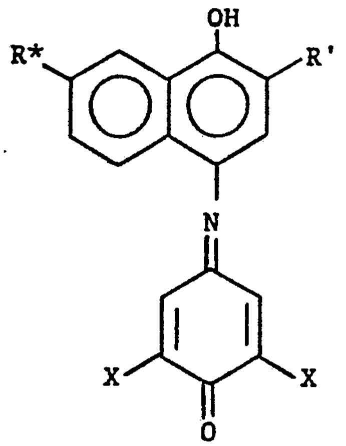

dans laquelle R' est H ou un groupe alkyle inférieur ayant de 1 à 4 atomes de carbone, de préférence méthyle, R* est H ou un groupe alkyle intermédiaire ayant de 5 à 15 atomes de carbone, de préférence n-décyle, et X est un halogène ou un groupe —CN, —SCN, —OCN, —$N_3$, —COZ, —COOZ, —CONHZ, —$CF_3$, —$CCl_3$, —$NO_2$, —$SO_2CF_3$, —$SO_2CH_3$, —$SO_2$—$C_6H_5$, —$SO_2C_6H_4CH_3$, —$SOC_6H_5$ ou —$SOCF_3$, dans lequel R est un groupe alkyle ou aryle.

8. Un procédé pour préparer un élément d'essai pour déterminer la présence d'un ion dans un échantillon d'essai aqueux, le procédé comprenant les étapes suivantes:

a) on forme un premier mélange homogène de la composition hydrophobe comprenant: un ionophore capable de former un complexe avec l'ion, un véhicule hydrophobe, une substance indicatrice capable de réagir avec le complexe de l'ionophore et de l'ion pour produire une réponse détectable; et d'un solvant organique;

b) on forme un second mélange homogène d'une substance tampon capable de donner un pH dans la gamme d'environ 5 à 10 et d'eau ou d'un solvant miscible avec l'eau ou leurs mélanges;

c) on imprègne dans une matrice de support poreuse avec l'un des premier et second mélanges;

d) on sèche la matrice imprégnée;

e) on imprègne la matrice de support poreuse avec l'autre des premier et second mélanges pour former une matrice deux fois imprégnée; et

f) on sèche la matrice de support deux fois imprégnée.

9. Le procédé selon la revendication 8, dans lequel le premier mélange contient en outre un polymère hydrophobe.

10. Le procédé selon les revendications 8 ou 9, dans lequel le second mélange contient en outre une substance mouillante et/ou un système éliminant les ions gênants.

11. Un procédé pour déterminer la présence d'un ion dans un échantillon d'essai aqueux, le procédé consistant à mettre en contact l'échantillon d'essai avec l'élément d'essai selon l'une quelconque des revendications 1 à 7 et à observer une réponse détectable.

PREFERRED EMBODIMENT:
POTASSIUM RESPONSIVE TEST DEVICE

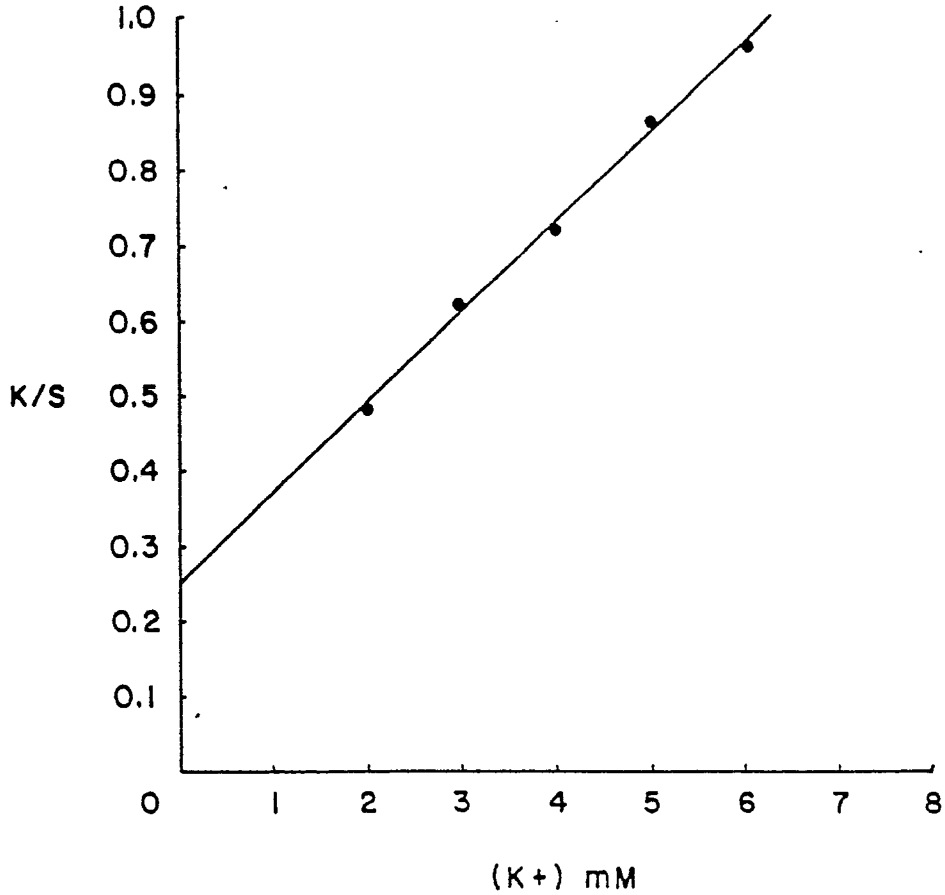


FIG. 1

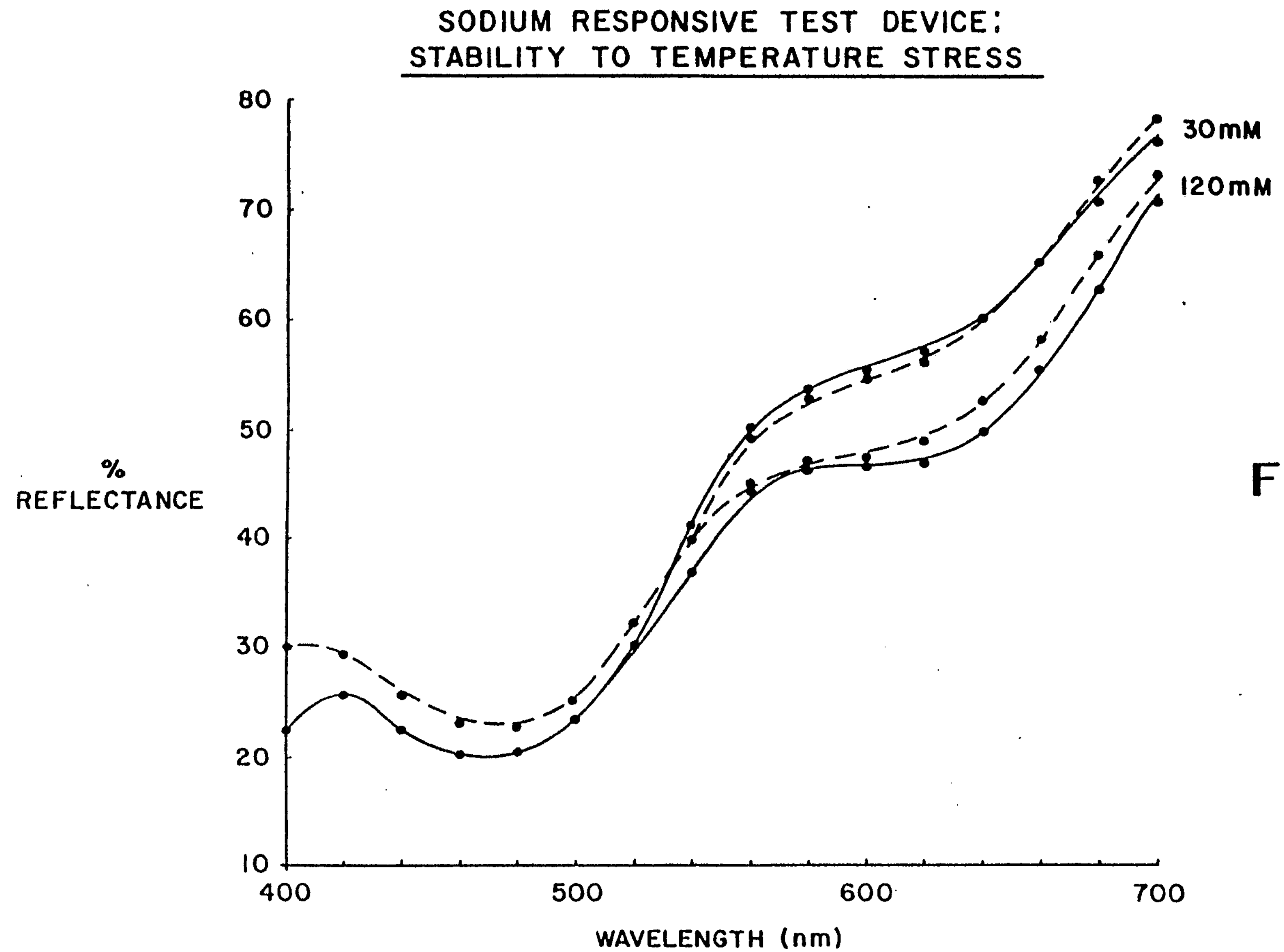
SODIUM RESPONSIVE TEST DEVICE:
STABILITY TO TEMPERATURE STRESS
% REFLECTANCE
80
70
60
50
40
30
20
10
400
500
600
700
WAVELENGTH (nm)
30mM
120mM

FIG. 2